# EUROPEAN PATENT SPECIFICATION

(11) **EP 4 175 688 B1**
(45) Date of publication and mention of the grant of the patent: **21.10.2026**
(21) Application number: 21735334.1
(22) Date of filing: 05.07.2021
(51) Int. Cl.: A61L 27/20, A61L 27/22, A61L 27/24, A61L 27/26, A61L 27/34, A61L 27/36, A61L 27/38, A61L 27/52, B33Y 70/00, B33Y 80/00

(54) **PRINTING METHOD AND SYSTEM FOR OBTAINING FREE-FORM WIDTH-CONTROLLED INDIVIDUAL BIOLOGICAL FIBERS**
DRUCKVERFAHREN UND -SYSTEM ZUR GEWINNUNG VON FREIFORM-BREITENKONTROLLIERTEN EINZELNEN BIOLOGISCHEN FASERN
PROCÉDÉ ET SYSTÈME D'IMPRESSION POUR L'OBTENTION DE FIBRES BIOLOGIQUES INDIVIDUELLES DE FORME LIBRE ET DE LARGEUR CONTRÔLÉE

(30) Priority: 03.07.2020 EP 20382597
(43) Date of publication of application: 10.05.2023
(73) Proprietor: Fundació Institut de Bioenginyeria de Catalunya (IBEC), 08028 Barcelona (ES); Institució Catalana De Recerca I Estudis Avançats (ICREA), 08010 Barcelona (ES)
(72) Inventor: SÁNCHEZ ORDOÑEZ, Samuel, 08010 Barcelona (ES); MESTRE CASTILLO, Rafael, 08028 Barcelona (ES); GUIX NOGUERA, María, 08028 Barcelona (ES); PATIÑO PADIAL, Tania, 08028 Barcelona (ES); FUENTES LLANOS, Judith, 08028 Barcelona (ES)
(74) Representative: Hoffmann Eitle
(86) International application number: PCT/EP2021/068502
(87) International publication number: WO 2022/003203

(56) References cited:
- WO-A1-2015/066705
- WO-A1-2017/187114
- WO-A1-2018/053565
- WO-A1-2018/055452
- WO-A1-2019/060518
- COSTANTINI MARCO ET AL: "Microfluidic-enhanced 3D bioprinting of aligned myoblast-laden hydrogels leads to functionally organized myofibersin vitroandin vivo", BIOMATERIALS, ELSEVIER, AMSTERDAM, NL, vol. 131, 23 March 2017 (2017-03-23), pages 98 - 110, XP029991706, ISSN: 0142-9612, DOI: 10.1016/J.BIOMATERIALS.2017.03.026
- KATJA HÖLZL ET AL: "Bioink properties before, during and after 3D bioprinting", BIOFABRICATION, vol. 8, no. 3, 23 September 2016 (2016-09-23), pages 032002, XP055599735, DOI: 10.1088/1758-5090/8/3/032002
- T. J. HINTON ET AL: "Three-dimensional printing of complex biological structures by freeform reversible embedding of suspended hydrogels", SCIENCE ADVANCES, vol. 1, no. 9, 23 October 2015 (2015-10-23), pages e1500758 - e1500758, XP055491252, DOI: 10.1126/sciadv.1500758
- HYUN-WOOK KANG ET AL: "A 3D bioprinting system to produce human-scale tissue constructs with structural integrity", NATURE BIOTECHNOLOGY, vol. 34, no. 3, 1 March 2016 (2016-03-01), us, pages 312 - 319, XP055556663, ISSN: 1087-0156, DOI: 10.1038/nbt.3413
- MESTRE RAFAEL ET AL: "3D Bioprinted Muscle-Based Bio-Actuators: Force Adaptability Due to Training", 7 July 2018, BIG DATA ANALYTICS IN THE SOCIAL AND UBIQUITOUS CONTEXT : 5TH INTERNATIONAL WORKSHOP ON MODELING SOCIAL MEDIA, MSM 2014, 5TH INTERNATIONAL WORKSHOP ON MINING UBIQUITOUS AND SOCIAL ENVIRONMENTS, MUSE 2014 AND FIRST INTERNATIONAL WORKSHOP ON MACHINE LE, ISBN: 978-3-642-17318-9, XP047478021

## Description

### FIELD

The present disclosure relates to a printing system for obtaining individual free-form width-controlled biological fibers, to the free-form individual fibers obtained thereby and to methods for obtaining these. The present disclosure also relates to a hybrid biocompatible machine or a biomimetic structure comprising one or more of said individual fibers, and to methods of manufacturing thereof.

### BACKGROUND

3D bioprinting is a technique that allows for printing of biological materials such as cell-laden hydrogels according to different designs, in order to mimic the 3D environment of biology such as native tissue. Two-dimensional cultures have been widely used in biological research, but there is an increasing understanding that some of the properties of biological materials might be completely different in native three-dimensional environments. 3D bioprinting offers an advantage over two-dimensional cultures by more closely approximating the native 3D environment of a biological material.

Several modes of 3D bioprinting have been proposed. Pneumatic extrusion-based bioprinting is the most widely used technique, and it is based on the extrusion of a cell-laden hydrogel (or any other polymer) through a thin nozzle by the application of pressure. This technique uses materials that have enough pseudoplastic or shear-thinning behaviour that decreases their viscosity when shear rate is increased (through the nozzle). This reduction of viscosity allows printing at lower pressures but also helps protect the cells from high shear stresses by decreasing the viscosity.

As one example, skeletal muscle tissue has an inherently three-dimensional architecture, composed of bundles of muscle fibers (myocytes), created after fusion of myoblasts during the differentiation process. Myocytes within the bundles are densely packed and highly aligned in order to achieve very efficient and longitudinal contractions. Inside each myocyte, groups of internal protein structures (myofibrils) are formed. These structures contain periodic units (sarcomeres), which are mainly composed of actin fibers and myosin, and have the ability of contracting the myocyte upon certain stimulus. Therefore, given the complex three-dimensionality of skeletal muscle tissue in its natural form, any tissue model that aims at recreating its complexity must present a three-dimensional conformation.

Mouse myoblasts have already been 3D-bioprinted embedded in bioinks and their differentiation induced in this environment to form multinucleated myotubes (H.-W. Kang, S. J. Lee, I. K. Ko, C. Kengla, J. J. Yoo, and A. Atala, A 3D bioprinting system to produce human-scale tissue constructs with structural integrity, Nature Biotechnology 34, 312 (2016); Mestre, R., Patiño, T., Barceló, X., Anand, S., Pérez-Jiménez, A., & Sánchez, S. (2019). Force modulation and adaptability of 3D-bioprinted biological actuators based on skeletal muscle tissue. Advanced Materials Technologies, 4(2), 1800631. Other approaches not based on 3D bioprinting, but mold casting techniques, have also been described for the obtaining of muscle tissue (R. Raman, C. Cvetkovic, and R. Bashir, A modular approach to the design, fabrication, and characterization of muscle-powered biological machines, Nature Protocols 12, 519 (2017)). Nonetheless, these techniques lack the versatility, automation and fast-prototyping capabilities of 3D bioprinting.

Moreover, in native tissue, each bundle of myocytes is further organized in fascia, or fascicles, surrounded by the perimysium, a layer of connective tissue (mainly collagen) that stabilizes and separates the bundles of muscle fibers, containing from 10 to 100 of them. This fascicle structure has not been completely mimicked yet with 3D bioprinting, as subsequent layers of 3D-bioprinted fibers fuse with each other to form an even wider construct. The closest analogs to these kinds of structures have relied on sacrificial molds and microfabrication techniques (D. Neal, M. S. Sakar, L.-L. S. Ong, and H. Harry Asada, Formation of elongated fascicle-inspired 3D tissues consisting of high-density, aligned cells using sacrificial outer molding, Lab Chip 14, 1907 (2014). Thus, there is a need to provide a 3D bioprinting method of individual fibers, e.g. myoblast cell-laden individual fibers, which do not significantly fuse to each other upon deposition in a multi-layer form.

Moreover, the thickness of the biological fibers that can be achieved with 3D bioprinting is difficult to control. As the oxygen diffusion limit within a tissue is around 200 µm, wider tissues can suffer from biocompatibility issues or low cell density in the inner parts of the 3D-printed hydrogel. This causes a great loss of efficiency, as the bundle of myotubes will not be densely packed, but actually depleted. For example, in the case of skeletal muscle tissue, the lower density of packing will diminish the total force per cross-sectional area that can be achieved. Thus, there is also a need to obtain thin, width controlled fibers.

For example, some printing systems manufacture fibers by extruding the liquid phase biomaterial from the bioprinter nozzle into a support bath filled with a cross-linking solution (US 10156560 B1; Hinton T.J. et al., Three-dimensional printing of complex biological structures by freeform reversible embedding of suspended hydrogels; Sci. Adv.; 1:e1500758 (2015)). In this system, fixation of the printed filament diameter is not simultaneous to printing but only occurs after deposition. Moreover, the type of cross-linking system which can be used is limited to chemical cross-linking.

The block co-polymer Pluronic^{™} F-127, has previously been used as a sacrificial ink for structures that need infilling or three-dimensional support to keep fiber separation before cross-linking (W. Wu, A. Deconinck, and J. A. Lewis, Omnidirectional printing of 3D microvascular networks, Advanced Materials 23, 178 (2011); Hyun-Wook Kang et al. A 3D bioprinting system to produce human-scale tissue constructs with structural integrity, Nature Biotechnology, vol. 34, no. 3, pages 312-319 (2016)).

In Hyun-Wook Kang et al. a Pluronic^{™} F-127 hydrogel was disposed following a specific pattern to support the 3D architecture of the dispensed cell-laden structures before crosslinking. After crosslinking of fibrinogen using thrombin, the uncrosslinked components (gelatin, HA, glycerol and Pluronic F-127) were washed out. This method thus uses a Pluronic^{™} F-127 hydrogel as a sacrificial material but does not enable to obtain free-form fibers since these will be disposed according to a predesigned pattern.

One of the most common strategies for obtaining free-form fibers with a controlled width is the use of co-axial printing of an alginate hydrogel, which is chemically cross-linked *in situ* during the extrusion process In particular, an alginate hydrogel is extruded from an internal nozzle whilst a cross-linking CaCl₂ solution is flowed out of the external needle (Ahn et al. A novel cell-printing method and its application to hepatogenic differentiation of human adipose stem cell-embedded mesh structures, Scientific Reports volume 5, 13427 (2015); WO 2015/066705 A1; Costantini Marco et al. Microfluidic-enhanced 3D bioprinting of aligned myoblast-laden hydrogels leads to functionally organized myofibersin vitro and in vivo, Biomaterials, vol. 13, pages 98-110 (2017); Katja Hölzl et al. Bioink properties before, during and after 3D bioprinting, Biofabrication, vol. 8, no. 3, page 032002 (2016); Colosi C, Shin SR, Manoharan V, Massa S, Costantini M, Barbetta A, Dokmeci MR, Dentini M, Khademhosseini A. Microfluidic Bioprinting of Heterogeneous 3D Tissue Constructs Using Low-Viscosity Bioink. Adv Mater. 28(4):677-84 (2016).

This system does not enable cross-linking by methods other than chemical cross-linking. Thus, it limits the nature of the printed hydrogel by requiring the presence of alginate, which is typically around 3% in the bioink composition. Some cells types, such as myoblasts or vascular cells, might not proliferate and/or differentiate properly in alginate due to the absence of cell-binding sites, and thus alginate is usually removed by a chelating agent (e.g. 20 mM EDTA) after bioprinting a cell-laden hydrogel and prior to cell culturing (Colosi C. et al. 2016). Moreover, the flow of the cross-linking solution from the external nozzle is difficult to control. Also, obstruction of the nozzle due to accumulation of cross-linked alginate at the exit is very common. Therefore, it is difficult to finely control the diameter of the printed fiber. Thus, there is a need in the art for a method to obtain free form fibers which do not contain or have a reduced percentage of alginate.

WO 2018/053565 A1 discloses a method for the biofabrication of free-form fibers using bio-printers with a dual chamber having two nozzles in a co-axial arrangement. As described in Example 4, the inner chamber had GelMa/HAMa hydrogel seeded with mesenchymal stem cells and the external chamber GelMa/HAMa hydrogel and 0.5 wt% photoinitiator VA-086. It further describes that upon extrusion the external UV-curable surface of the fiber was exposed to a UV-lamp creating an external shell. Accordingly, this method requires presence of a UV-curable polymer and a photoinitiator (e.g. VA-086) in the external bioink to induce crosslinking upon radiation. This method further requires exposure to an uv-source upon extrusion. It should further be noted that the external shell is not removed after cross-linking thus adding width to the final fiber.

WO 2015/066705 A1 describes a method which aims to obtain individual width-control individual free-form fibers without the need of chemical cross-linking or uv cross-linking which is based in the inclusion of a thermoreversible polymer (Pluronic^{™} F-127) for thickening the bioink when printed directly in the subject's tissue. This method has the limitation that fixation of the fiber structure and width only occurs upon direct deposition into a subject's skin. Thus, fixation of the structure only occurs after deposition. Moreover, it does not provide free-form individual fibers with a controlled width which can be manipulated *in vitro* (e.g. by inducing differentiation of the embedded cells) prior to deposition in a subject.

Accordingly, despite recent advancements, there is a need for a universal 3D bioprinting system and method for the fabrication of thin, homogeneous and width-controlled free-form fibres of virtually any hydrogel.

Moreover, there is a need for the fabrication of bundle-like skeletal muscle fibres that could be potentially assembled to form fascicle-like structures, morphologically, physiologically and functionally similar to those present in native tissue.

### SUMMARY

The inventors have provided a universal method for the fabrication of thin, homogeneous and width-controlled free-form fibres of virtually any hydrogel by inventively separating the step of fixating the 3D-printed fiber structure from the hydrogel polymer cross-linking step. In particular, the method of the invention enables the fabrication of multi-layer tissue constructs without significant fusion of adjacent fibers.

This effect is based on a co-axial method and printing system, wherein a physical confinement of the fibers occurs upon extrusion, more specifically by coating the extruded hydrogel (in the inner nozzle) with a polymeric composition (e.g. Pluronic^{®} F-127) in a gel state (in the outer nozzle). As a result, the structure of the fiber is fixed immediately upon extrusion, thus improving width control by avoiding the potential expansion of the hydrogel and preventing significant fusion of adjacent fibers when these are printed in a superposed manner to form a multi-layer tissue construct. The hydrogel can subsequently be cross-linked by any known method, e.g, by chemical, thermal or enzymatic cross-linking or by cross-linking induced by exposure to UV light, and then the coating polymer can be removed (e.g., Pluronic^{®} F-127 is easily removed with a cold aqueous solution). Said external composition comprises preferably a thermoreversible gelation sacrificial polymer (e.g. Pluronic^{®} F-127). Further to the removal of the external polymer shell, the diameter of the fiber obtained by the method of the invention is that of the inner hydrogel.

Furthermore, this method offers high versatility since the hydrogel composition is not limited (conversely to a co-axial printing with a CaCl₂ solution in the outer nozzle which requires a hydrogel comprising alginate) and can be adjusted for specific needs of cell lines or even different cross-linking methods.

If a finer control of the fibers width is desired the physical confinement and chemical cross-linking can be combined (referred herein as chemically assisted physical confinement), for instance by incorporating a chemical cross-linking agent into the coating polymeric composition (e.g. CaCl₂ for alginate hydrogels).

The invention is defined by the set of claims.

According to a first aspect of the invention, there is provided a method for obtaining one or more individual free-form fibers of biocompatible hydrogels with a predefined diameter, wherein said method comprises the use of a printing system comprising at least a first nozzle and a second nozzle surrounding the first nozzle (e.g., a co-axial nozzle), wherein said method comprises the following steps: a) providing a printable biocompatible hydrogel in the first nozzle; b) providing a printable composition comprising a non-toxic thermoreversible gelation polymer in the second nozzle, wherein said composition comprises or consists of a poloxamer-based thermoreversible gel, and wherein said composition comprises poloxamer 407; c) extruding the biocompatible hydrogel in a) and the composition in b) simultaneously through the nozzles, wherein the composition in b) coats in a solidified state the extruded biocompatible hydrogel; d) submitting the obtained one or more individual fibers to a cross-linking treatment; e) removing the composition in b) from the external surface of the deposited one or more fibers; wherein
(i) the biocompatible hydrogel comprises a substance which crosslinks upon exposure to a cross-linking agent, and wherein the cross-linking treatment in step d) comprises exposing the obtained fiber to a cross-linking agent; optionally wherein the polymer composition in b) comprises a cross-linking agent and steps c) and d) are performed simultaneously;
(ii) the biocompatible hydrogel comprises a substance which crosslinks upon exposure to UV light, and wherein the cross-linking treatment comprises applying UV light to the obtained fiber; and/or
(iii) the biocompatible hydrogel comprises a substance which crosslinks upon heating, and wherein the cross-linking treatment comprises heating the obtained fiber.

The present disclosure further relates to the fibers obtained or obtainable by a method of the first aspect wherein, said fiber is the one obtained or obtainable after steps a) to c). Thus, in a second aspect the invention relates to an individual free-form fiber of a biocompatible hydrogel wherein said biocompatible hydrogel is as defined in the first aspect, wherein said fiber is coated with a composition comprising a thermoreversible gelation polymer in gel state and wherein said composition comprises poloxamer 407.

According to a third aspect of the invention, there is provided a method of manufacturing a hybrid biocompatible machine or a biomimetic structure comprising one or more individual fibers of biocompatible hydrogels as defined in the second aspect, wherein said method comprises a step of depositing said one or more individual fibers on or within said machine or biomimetic structure or assembling these forming a biomimetic structure, preferably wherein said fibers form a multi-layer construct.

According to a fourth aspect of the invention, there is provided a hybrid biocompatible machine comprising individual fibers as defined in the second aspect, e.g., comprising skeletal muscle myotubes.

According to a fifth aspect of the invention, there is provided a biomimetic structure comprising individual fibers as defined in the second aspect, e.g., comprising skeletal muscle myotubes. In a preferred embodiment, these fibers form a multi-layer tissue construct.

In some embodiments, the invention relates to one or more individual fibers of biocompatible hydrogels of the second aspect, or a biomimetic structure of the fifth aspect, wherein the individual fibers comprise skeletal muscle myotubes.

According to a sixth aspect of the invention, there is provided a printing system for obtaining one or more free-form individual fibers of biocompatible hydrogels with a predefined diameter, wherein the printing system comprises: at least a first nozzle and a second co-axial nozzle surrounding the first nozzle; a source of a printable biocompatible hydrogel as defined in the first aspect connected to the first nozzle; and a source of a non-toxic thermoreversible gelation polymer composition connected to the second nozzle, wherein said composition comprises or consists of a poloxamer-based thermoreversible gel, and wherein said composition comprises poloxamer 407; wherein the printing system is configured to extrude the biocompatible hydrogel and the non-toxic thermoreversible gelation polymer simultaneously through the nozzles, such that when extruded, the non-toxic polymer coats in a gel state the extruded biocompatible composition.

### BRIEF DESCRIPTION OF THE DRAWINGS

To enable better understanding of the present disclosure, and to show how the same may be carried into effect, reference will now be made, by way of example only, to the accompanying schematic drawings, in which:
FIG. 1 shows a cross-sectional side view of a printing system according to one or more embodiments shown and described herein;
FIG. 2A shows a cross-sectional side view of an individual fiber obtained by the printing system of FIG. 1 according to one or more embodiments shown and described herein;
FIG. 2B shows a cross-sectional side view of another individual fiber obtained by the printing system of FIG. 1 according to one or more embodiments shown and described herein;
FIG. 3 shows a cross-sectional side view of a printing system according to one or more embodiments shown and described herein;
FIG. 4 shows a graph illustrating cell viability after 24 hours in fibers printed by various printing systems according to one or more embodiments shown and described herein. In particular, when using cylindrical thin, cylindrical wide and conical nozzles. Cell viability after 24 h shows that conical needles provide less cell damage due to less amount of shear stress. Letters indicate equal significance levels with p < 0.05 (one-way ANOVA followed by Tukey's HSD);
FIG. 5 shows imaged fibers obtained with a printing system according to one or more embodiments shown and described herein ("with pluronic" images A to C) as compared to fibers extruded from a printing system without an outer non-toxic polymer provided ("without pluronic" images D to F). FIG.5 A and D are bright field images, it can be observed that the tissue is thicker without pluronic (D); Fluorescence images of the live/dead assay (B and E: ALIVE cells & C and F: DEAD cells) show a greater quantity of dead cells after 24 h for the printing without pluronic.

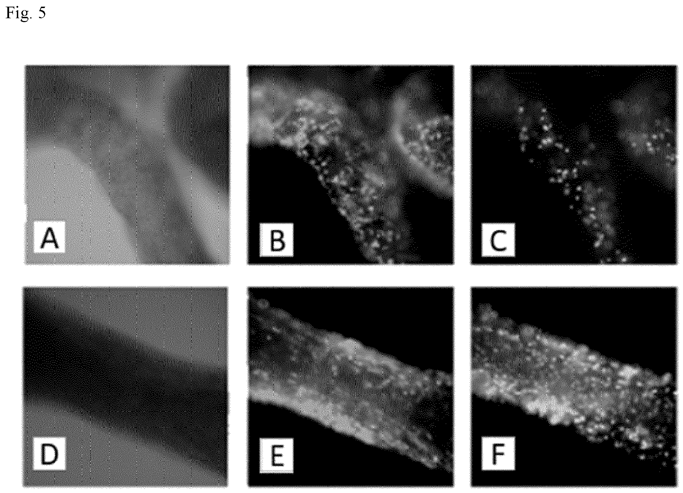

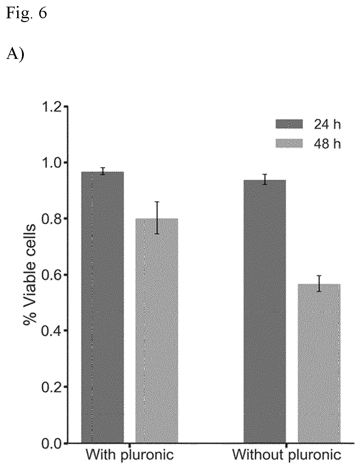

FIG. 6 A) is a graph illustrating the cell viability of the fibers shown in FIG 5 printed by a printing system according to one or more embodiments shown and described herein (with pluronic) as compared to fibers extruded from a printing system without an outer non-toxic polymer provided (without pluronic). Besides having similar viability after 24 h, there is a higher decrease of variability after 48 h for the case without pluronic confinement; B) shows the rheological characterization of Pluronic^{®} F-127 at 35% wt/v at two different temperatures. On the left, flow ramp showing the shear stress vs shear rate plot, and on the right viscosity vs shear rate, calculated as the slope of the curves on the left-hand figure. As an inset, a zoom-in of the shear stress vs shear rate plot at 4 °C. At low temperature, Pluronic behaves as a Newtonian liquid with constant viscosity (inset). At room temperature, it shows a strong shear-thinning behavior (right).
FIG. 7 shows a number of graphs illustrating possible combinations of materials in the biocompatible hydrogel and the non-toxic polymer to achieve a homogeneous individual fiber from the printing systems according to one or more embodiments shown and described herein. The preferred range of concentrations for obtaining homogeneous fibers is indicated by the region marked "X".
FIG. 8 shows a number of graphs illustrating possible combinations of materials that provide sufficient cell attachment sites from the printing systems according to one or more embodiments shown and described herein. The preferred range of concentrations for obtaining homogeneous fibers is indicated by the region marked "X".
FIG. 9A shows a side view of a hybrid biocompatible machine according to one or more embodiments shown and described herein.
FIG. 9B shows a top view of the hybrid biocompatible machine of FIG. 9A, according to one or more embodiments shown and described herein.
FIG. 10A shows an image of a biological fiber obtained with the chemically-assisted physical confinement strategy described in Example 1.1, after differentiation of myoblasts into myotubes. The biocompatible hydrogel comprised: 0.5% (wt/v) alginate, 3% (wt/v) gelatin 20 mg/mL fibrinogen and myoblasts at a density of 5 million cells/mL, the polymer composition in the outer shell comprised poloxamer 407 at 33% (wt/v) and 300 mM CaCl₂.
FIG. 10B shows a graph illustrating observed contractions in the biological fiber of FIG. 10A.
FIG. 10C shows an immunostaining image of the biological fiber of FIG. 10A after differentiation of myoblasts into myotubes.
FIG. 11A shows an image of a biological fiber obtained with the physical confinement method described in Example 1.2., after differentiation of myoblasts into myotubes. The biocompatible hydrogel comprised gelatin at 5% (wt/v) with fibrinogen at 20 mg/mL and myoblasts at a density of 5 million cells/mL and the polymer composition in the outer shell comprised poloxamer 407 at 33% (wt/v).
FIG. 11B shows a graph illustrating observed contractions in the biological fiber of FIG. 11A.
FIG. 11C shows an immunostaining image of the biological fiber of FIG. 11A after myoblast cell differentiation into myotubes.
FIG. 12 shows a graph illustrating the width of fibers obtainable by varying the pressure applied to an inner nozzle of a printing system according to one or more embodiments shown and described herein, more specifically fibers obtained by the chemically-assisted physical confinement (points 3 - 4) and physical confinement (points 1 - 2) methods of Examples 1 and 2, respectively. N = 8-12.
FIG. 13A to 13D show bright field microscopy images of biological fibers obtained by a printing system according to one or more embodiments shown and described herein, each image corresponding to a point on the graph of FIG. 12.
FIG. 14. Fibre width after 3D bioprinting with the pluronic-assisted co-axial system and normal (single-nozzle) printing of 1 and 3 layers. Letters indicate equal significance levels with p < 0.05 (one-way ANOVA followed by Tukey's HSD).
FIG. 15. Standard deviation of a set of 5-7 fibres printed with the pluronic-assisted co-axial system and normal (single-nozzle) printing of 1 and 3 layers.
FIG. 16. Force generated by skeletal muscle tissue constructs comprising 5 layers, 3D bioprinted with the pluronic-assisted co-axial system or a normal (single-nozzle) system after 4 days (D4) or 9 days (D9) of differentiation. Letters indicate equal significance levels with p < 0.05 (two-way ANOVA followed by Tukey's HSD).
FIG. 17 A) Bright field microscopy image of a skeletal muscle tissue construct after 9 days of differentiation, 3D bioprinted with the pluronic-assisted co-axial method obtaining 3 layers. B) Intensity projection along the yellow line in 17A. Changes in intensity indicated by the dashed line show inhomogeneities in the tissue, which are consistent with separation points of the three layers.
FIG. 18. A) Bright field microscopy image of a skeletal muscle tissue construct after 9 days of differentiation, 3D bioprinted with a normal (single-nozzle) method obtaining 3 layers. B) Intensity projection along the yellow line in 18A. No changes in intensity indicate that the layers are completely fused with each other.
FIG. 19. Representative examples of skeletal muscle tissue constructs 3D bioprinted with the pluronic-assisted co-axial method, with dashed lines representing separation of the tissue stripes, which were indicated by the changes in bright field light intensity, A) for 3 layers B) for 4 layers and C) for 6 layers.

### DETAILED DESCRIPTION

As used herein, a "printable" substance is any substance which is extrudable from a nozzle. Preferred biopolymers for 3D bioprinting are those presenting pseudoplastic or shear-thinning behaviour that decreases their viscosity when shear rate is increased (through the nozzle). Due to the shear stress felt at the nozzle, the free chains of an uncrosslinked polymer are free to re-orient longitudinally in the same direction. At this point, the viscosity of the material decreases locally, allowing a smooth printing, and, after deposition, the chains take again random orientations, increasing its viscosity and retaining the printed shape. Preferably the viscosity of the hydrogels formed by suitable polymers during extrusion are within the range of 30 mPa.s to 6x10⁷ mPa.s. Measurements of (dynamic) viscosity of the hydrogels can be done by using any controlled-stress rheometer and performing a flow ramp measurement of shear stress vs shear rate, preferably at 25 °C (room temperature) or by setting a temperature that defines the bioprinting environment. From this shear stress vs shear rate plot, the viscosity of the material at a frequency of 1 Hz is taken as representative of the dynamic viscosity of the material.

As used herein, the term "non-toxic" is to be understood as biocompatible.

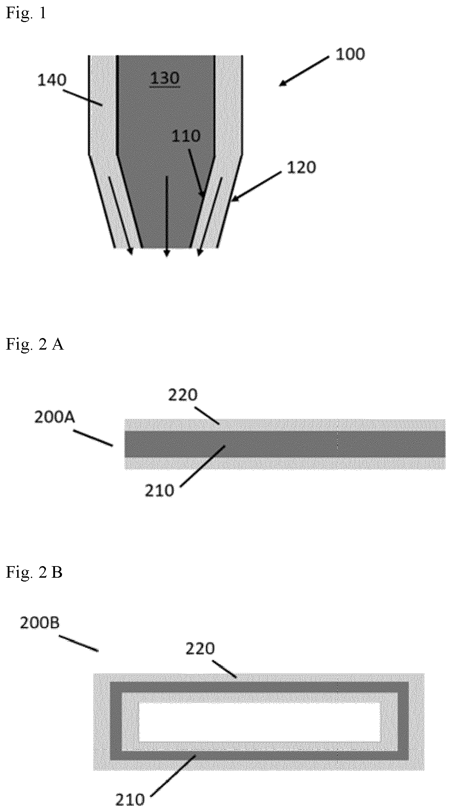

FIG. 1 shows a cross-sectional side view of a printing system 100 according to one or more embodiments. The printing system 100 comprises a first nozzle 110 and a second co-axial nozzle 120 surrounding the first nozzle 110. A printable biological composition, and more specifically a biocompatible hydrogel 130 is provided in the first nozzle 110 and a printable composition 140 comprising one or more non-toxic polymers is provided in the second nozzle 120.

The printing system 100 is configured so that the biocompatible hydrogel 130 and the printable composition 140 are extruded from the first nozzle 110 and second nozzle 120 simultaneously, such that the printable composition 140 coats the biocompatible hydrogel 130. The printable composition 140 is configured to coat the biocompatible hydrogel 130 in a solidified state. As a result, the printing system 100 prints individual fibers of biocompatible hydrogels which are coated in a solidified composition 140 comprising a non-toxic polymer.

Any printable composition which is non-toxic and configured to coat the extruded biocompatible hydrogel 130 in a solidified state may be used for the composition 140.

The printable composition 140 as defined under the first aspect comprises one or more non-toxic thermoreversible gelation polymers, also referred in the art as thermoresponsive sacrifical polymers. In particular, a polymer which is solid (also referred as gel state) at a temperature between 10 °C - 40 °C is preferred, as this allows the extruded fibers to be incubated at a particular temperature (for example for temperature-assisted crosslinking) whilst the composition 140 is in a solid state, thereby maintaining the shape of the fiber whilst being incubated.

More specifically, the composition 140 is a poloxamer-based thermoreversible gel. Poloxamers or Pluronics^{®} are a class of water-soluble non-ionic triblock copolymers formed by polar (poly ethylene oxide) and non-polar (poly propylene oxide) blocks which confer amphiphilic and surface active properties to the polymers. Their aqueous solutions undergo sol-to-gel transition with increasing the temperature above a lower critical gelation temperature (LCGT); moreover, the coexistence of hydrophilic and hydrophobic monomers into block copolymers allows the formation of ordered structures in solution, the most common of these being the micelles. A variety of poloxamers is available on the market, differing on the molecular weight of the building blocks and on the hydrophobic-hydrophilic ratio, allowing the preparation of thermosensitive hydrogels with different properties, e.g., in terms of critical gelation concentration (CGC) and gelation time at physiological condition. The most significant physical properties of most common poloxamers are described in Russo E. and Villa C. Poloxamer Hydrogels for Biomedical Applications, Pharmaceutics 2019, 11, 671. Other sacrificial thermoreversible gelation polymer-based compositions are known in the art, such as sacrificial sugar structures, e.g., the so-called carbohydrate glass, comprising a mixture of glucose, sucrose and dextran (86 kDa) (Miller, J., Stevens, K., Yang, M. et al. Rapid casting of patterned vascular networks for perfusable engineered three-dimensional tissues. Nature Mater 11, 768-774 (2012); Bellan, L. M. et al. Fabrication of an artificial 3-dimensional vascular network using sacrificial sugar structures. Soft Matter 5, 1354-1357 (2009); compositions comprising gelatin, such as a composition comprising 9% methylcellulose and 5% gelatin (Dranseikiene, D., Schrüfer, S., Schubert, D.W. et al. Cell-laden alginate dialdehyde-gelatin hydrogels formed in 3D printed sacrificial gel. J Mater Sci: Mater Med 31, 31 (2020); Golden, A. P. & Tien, J. Fabrication of microfluidic hydrogels using molded gelatin as a sacrificial element. Lab Chip 7, 720-725 (2007)), or NiPAAM (Tebong Mbah V, et al. A Sacrificial PLA Block Mediated Route to Injectable and Degradable PNIPAAm-Based Hydrogels. Polymers (Basel) 12(4):925 (2020); Lee JB, et al. Development of 3D Microvascular Networks Within Gelatin Hydrogels Using Thermoresponsive Sacrificial Microfibers. Adv Healthc Mater. 5(7):781-5 (2016)).

In particularly preferred embodiments, said printable composition 140 is a poloxamer hydrogel in water-based media, and comprises poloxamer 407 (CAS number: 691397-13-4; e.g., Pluronic^{®} F-127). For instance, poloxamer 407 may be at a concentration of at least 25%, preferably 25-45 % (wt/v), more preferably of 30-40% (wt/v), more preferably of 33-37 % (wt/v). It is noted that an even higher concentration of poloxamer 407 may be possible, so long as the printing system is able to apply the required pressure for extrusion.

The biocompatible hydrogel 130 may comprise one or more synthetic or natural biopolymers in an aqueous media. Natural biopolymers would include any polysaccharides and/or proteins which provide a printable composition. The biocompatible hydrogel 130 may comprise for instance one or more of a decellularized extracellular matrix (ECM), alginate, modified alginate comprising inserted cell attachment sites, gelatin, fibrinogen, laminin, collagen, or other ECM proteins, hyaluronic acid, as well as any of these modified with acrylamide groups, such as gelatin methacrylate (GelMA), alginate methacrylate (AlgMA) collagen methacrylate (ColMA), or hyaluronan methacrylate (HA-MA), chitosan, gellan gum, xanthan gum, agarose, poly(ethylene glycol) diacrylate (PEGDA), N-Isopropylacrylamide (NIPAM), or nanocellulose. Some examples of biocompatible hydrogels are described in Table 1. Preferred embodiments comprise or consist of (i) fibrinogen and gelatin, (ii) fibrinogen, a decellularized ECM, ECM-based hydrogels or other cell matrices (e.g. Matrigel^{®}) and gelatin, (iii) a decellularized ECM, ECM-based hydrogels or other cell matrices (e.g. Matrigel^{®}), alginate and gelatin, (iv) fibrinogen, gelatin and alginate, (v) fibrinogen, a decellularized ECM, ECM-based hydrogels or other cell matrices (e.g. Matrigel^{®}), gelatin and alginate, (vi) fibrinogen, gelMA and alginate or (vii) fibrinogen, a decellularized ECM, ECM-based hydrogels or other cell matrices (e.g. Matrigel^{®}), gelMA and alginate. In some instances, this biocompatible hydrogel 130 may be in nanostructured form further to the addition of nanocomposites. In preferred embodiments, optionally in combination with any of the embodiments described herein, the biocompatible hydrogel 130 does not comprise alginate. In other embodiments, optionally in combination with any of the embodiments described herein, the biocompatible hydrogel 130 does not comprise acrylate polymers (such as GelMA, AlgMA, ColMA or HA-MA) or another substance which crosslinks upon exposure to UV light.

The biocompatible hydrogel 130 may also comprise any living cells. The living cells can optionally range from about 5 million cells/mL to about 20 million cells/mL. Thus, it is contemplated that the cell concentration in the biocompatible hydrogel 130 can optionally be about 5 million cells/mL, about 10 million cells/mL, about 15 million cells/mL or about 20 million cells/mL. They type of cells included in the hydrogel can be selected, as appropriate, depending on the purpose. **In** preferred embodiments, these cells are animal cells, preferably these cells are mammalian cells. For example, these mammalian cells may be selected from a human, mouse, rat, guinea pig, dog, cat, cow, pig, sheep, horse, bear, and so on. **In** a preferred embodiment, said cells are mouse or human cells. The type of animal cells (e.g., human-derived cells) may be any of pluripotent cells, somatic stem cells, progenitor cells, and mature cells. Examples of the pluripotent cells that can be used herein include ES cells, GS cells, and iPS cells. Examples of the somatic stem cells that can be used herein include mesenchymal stem cells (MSC), hematopoietic stem cells, and neural stem cells. Examples of the progenitor cells and mature cells that can be used herein include cells derived from the skin, dermis, epidermis, muscle, cardiac muscle, nerve, bone, cartilage, endodermis, brain, epithelium, heart, kidney, liver, pancreas, spleen, oral cavity, cornea, or hair. Examples of the human-derived cells that can be used herein include ES cells, iPS cells, MSC, chondrocytes, osteoblasts, osteoprogenitor cells, mesenchyme cells, myoblasts, cardiac muscle cells, nerve cells, hepatic cells, beta cells, fibroblasts, corneal endothelial cells, vascular endothelial cells, corneal epithelial cells, and hematopoietic stem cells. For therapeutic purposes, the origin of cells may be either autologous or allogeneic.

After 3D bioprinting, the one or more individual fibers obtained further to extruding the biocompatible hydrogel 130 coated by the composition 140 in a gel state are submitted to a cross-linking treatment and subsequently the composition 140 is removed from the external surface of the deposited one or more fibers as described herein below. When the biocompatible hydrogel 130 is a cell-laden hydrogel, further to the fiber cross-linking and removal of the composition 140, the method of the invention may further comprise a culturing step wherein the fiber or fiber construct is cultured for cell growth and/or cell differentiation purposes. Cell growth and differentiation culture media are well known in the art and the person skilled in the art would know the more appropriate medium depending on the cell type, see for instance Yao, Tatsuma, and Yuta Asayama. "Animal-cell culture media: History, characteristics, and current issues." Reproductive medicine and biology 16.2 (2017): 99-117.

In a preferred embodiment, these cells are myoblasts (e.g., C2C12 myoblasts). Skeletal muscle myoblasts can differentiate into myotubes, complex cellular structures composed of several fused myoblasts with contractile abilities. For instance, the fiber or construct can be incubated in differentiation medium, such as the DM medium described in Example 2, which promotes cell differentiation into myotubes. The myoblasts may be cultured in the differentiation medium for 5-7 days, although more days may be preferred to achieve full maturation, preferably the cells are cultured for a total period of 7 to 14 days. In preferred embodiments, myotubes are aligned in the direction of the 3D bioprinted fibers, which is a necessary condition for maximum force generation.

Without willing to be bound by theory, 3D bioprinting can further help in the alignment of fibers due to the shear stress at the nozzle. This stress aligns the hydrogel fibers which in turn helps in the alignment of the myotubes, as they tend to follow topographical cues.

FIG. 2A shows a cross-sectional side view of an individual non-hollow fiber 200A obtained by the printing system 100 of FIG. 1. The individual fiber 200A comprises a non-hollow inner fiber 210 of the biocompatible hydrogel, and a solidified outer coating 220 of the composition 140 containing the non-toxic polymer which coats the inner fiber 210. The inner fiber 210 is provided with physical support by the outer coating 220, preventing the inner fiber 210 from losing its shape over time.

FIG. 2B shows another cross-sectional side view of an individual fiber 200B obtained by the printing system 100 of FIG. 1. Like in the example of FIG. 2A, the individual fiber 200B comprises an inner fiber 210 of the biocompatible hydrogel, and a solidified outer coating 220 of the composition 140 containing the non-toxic polymer. However, the individual fiber 200B has been printed in a closed loop.

The diameter of the biocompatible hydrogel obtained will depend on the diameter of the first nozzle 110, as well as the relative rates of extrusion of the biocompatible hydrogel 130 and the printable composition 140. The pressure applied to each nozzle may be independently controllable in order to control the diameter of the biocompatible hydrogel that is printed. For example, if the pressure applied to the first nozzle 110 is increased relative to the pressure applied to the second nozzle 120, the extrusion rate of the biocompatible hydrogel 130 will be greater and thus the coated fiber will have a greater diameter.

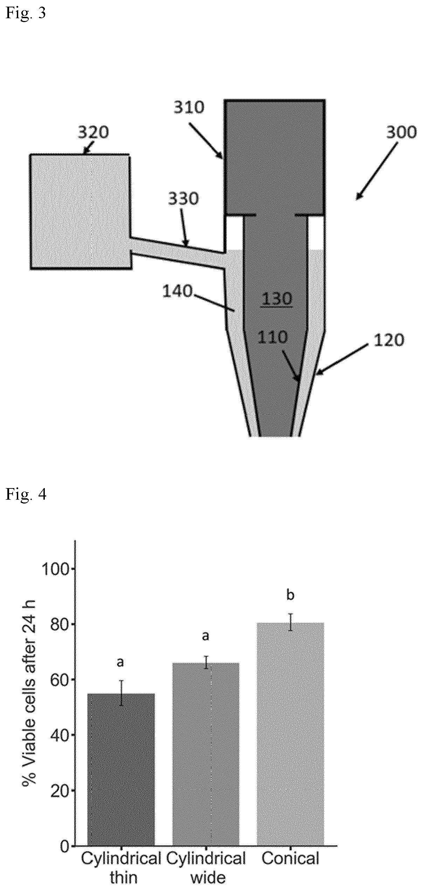

FIG. 3 shows a cross-sectional side view of a printing system 300 according to one or more embodiments. As in the case of FIG. 1, the printing system 300 comprises a first nozzle 110 and a second nozzle 120 surrounding the first nozzle 110. A printable biocompatible hydrogel 130 is provided in the first nozzle 110 and a printable composition 140 comprising a non-toxic polymer is provided in the second nozzle 120. The biocompatible hydrogel 130 is provided to the first nozzle 110 by a first cartridge 310 which contains a source of the biocompatible hydrogel 130, and which is in fluidic communication with the first nozzle 110. The composition 140 is provided to the second nozzle 120 by a second cartridge 320, which contains a source of the composition 140 and is fluidically connected to the second nozzle 120 via a conduit 330 extending laterally into the second nozzle 120.

As illustrated in FIG. 3, the conduit 330 may extend into the second nozzle 120 at an acute angle to the nozzle (i.e. at an angle less than 90 degrees), which may help prevent the conduit 330 and second cartridge 320 from contacting a vessel into which the fibers are printed (for example a petri dish). Alternatively, or additionally, the conduit 330 may comprise a flexible material such that the second cartridge 320 is moveable to avoid contact with a vessel into which the fibers are printed.

It is noted that the interior of the first nozzle 110 in FIGs. 1 and 3 is conical. In other embodiments, the interior of the first nozzle 110 may be cylindrical. FIG. 4 shows the percentage of cell viability after 24 hours of an extruded biocompatible hydrogel when a thin cylindrical nozzle (of approximately 700µm diameter), wide cylindrical (of approximately 840 µm diameter) and a conical nozzle (of approximately 200 µm diameter) is used for the same biocompatible hydrogel. The cell viability of the conical nozzle was higher than the cylindrical nozzles, even with a smaller diameter, as conical nozzles provide better stress profiles for bioprinting, by reducing the amount of shear stress applied to the biocompatible hydrogel during extrusion, which results in the greater cell viability. When cylindrical nozzles are used, the length of the cylindrical nozzles may be 25 mm or more.

The first nozzle 110 may be made of any suitable material, such as a metal or plastic. The first nozzle 110 is preferably made of plastic as this further reduces the amount of shear stress applied to the biocompatible hydrogel during extrusion, which improves the viability of the extruded hydrogel.

The diameter of the hole of the first nozzle 110 is preferably from 100 µm to 800 µm, more preferably from 150 µm to 400 µm, such as 200 µm, 250 µm, 300 µm or 350 µm, even more preferably about 200 µm. The diameter of the hole of the second nozzle 120 is preferably from 0.5 mm or 1 mm to 5 mm, more preferably from 0.5 mm to 1 mm, even more preferably about 800 µm. In a preferred embodiment the first nozzle has a diameter of 200 µm and the second nozzle has a diameter of 800 µm.

In particular, when a predetermined pressure is applied to the first nozzle, the one or more fibers have a predetermined diameter which may be diameter with a standard deviation of 20% or less, such as 15% or less, 10% or less, 9% or less, 8% or less, 7% or less, 6% or less, and more preferably 5% or less with respect to the mean diameter of the fiber(s). In preferred embodiments, the standard deviation is of 10% or less.

Said predefined diameter may alternatively or in addition also be a mean diameter with a deviation of 50% or less, 40% or less, 30% or less, 20% or less, preferably 10% or less, more preferably 5% or less with respect to a target diameter, preferably wherein said target diameter is the diameter of the inner nozzle (e.g. ±20 µm for a first nozzle 110 with 200 µm diameter), preferably 5% or less (e.g. ±10 µm for a first nozzle 110 with 200 µm diameter).

In preferred embodiments, optionally in combination with any of the embodiments described herein, the fibers have a mean diameter from 160 µm to 240 µm, preferably from 180 µm to 220 µm, more preferably from 190 µm to 210 µm. The diameter of the fiber may be measured at room temperature (e.g. at 25°C) using bright field microscopy. For instance it made be measured by drawing a line with the "measure" tool of ImageJ software (ImageJ, U. S. National Institutes of Health, Bethesda, Maryland, USA, https://imagej.nih.gov/ij/, 1997-2018.) used in the Examples.

FIG. 5 shows imaged fibers obtained with a printing system according to one or more embodiments (images A to C) as compared to fibers extruded from a printing system without an outer non-toxic polymer provided (images D to F). Images A and D show a simple image of the produced fibers. Images B and E show fluorescence images of live cells in the fibers. Images C and F show fluorescence images of dead cells in the fibers.

For the fibers shown in FIG.5 A to C, a printing system such as that shown in FIGs. 1 and 3 was used, with a composition containing poloxamer 407 (e.g., Pluronic^{®} F-127) used as the printable composition 140. For the fibers shown in FIG.5 D to F, the same biocompatible hydrogel was printed without an outer layer such as disclosed herein. Upon comparison of images A and D it is clear that the extrusion of the biocompatible hydrogel with an outer non-toxic polymer coating (image A) maintains a smaller width of the fibers, as the solidified non-toxic polymer coating confines the biocompatible hydrogel in place. On the other hand, without this outer coating the biocompatible hydrogel is not confined and so the thickness of the fibers obtained without the coating is larger, as shown in image D. As less oxygen and nutrients are able to diffuse through the wider fibers obtained without the outer polymer coating, it is observed in image F that there are a greater number of dead cells as compared to image C.

FIG. 6A shows a graph illustrating this effect. As can be seen, the fibers obtained with the polymer coating still had 80% viable cells after 24 hours, whereas the fibers without the polymer coating had less than 60% viable cells.

It has therefore been observed that the printing systems and methods disclosed herein increase the controllability of the width of the extruded fibers, and further increase cell viability.

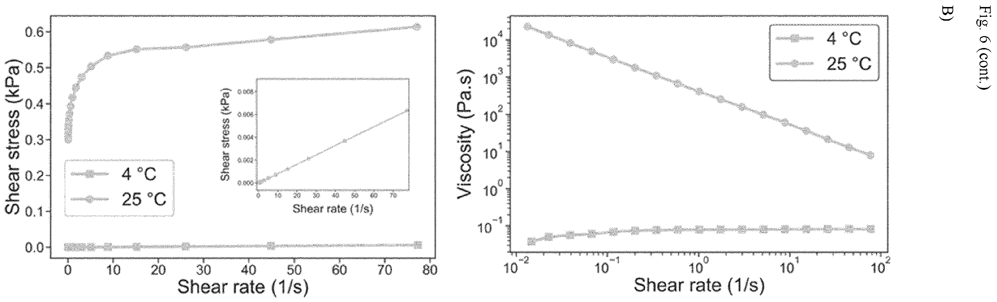

In the fibers obtained in FIG.5 A to C, a printable composition 140 comprising poloxamer 407 (e.g., Pluronic^{®} F-127) was used. Poloxamer 407 is particularly useful due to its thermoreversible properties. Using rheological characterisation methods, it has been observed that it behaves as a Newtonian liquid a low temperature (e.g. at 4 °C), and at room temperature (20 °C to 25 °C) shows a strong shear-thinning effect, meaning it has smooth printability whilst also retaining its shape once printed. The polymer may therefore be used as the printable composition 140 and may also be easily removed from the fibers by a wash with a cold aqueous composition such as water or phosphate buffered saline (i.e. at a temperature between 0 °C and 4 °C), see FIG. 6B.

As described previously, the printing systems and methods disclosed herein may be used to obtain one or more individual fibers of biocompatible hydrogels having a predefined diameter, by simultaneously extruding the biocompatible hydrogel 130 and the printable composition 140 such that the printable composition 140 coats in a solidified state (also referred herein as gel state) the extruded biocompatible hydrogel 130. The fibers obtained by the printing systems and methods described herein are characterized by being free-form fibers.

Once the one or more fibers are extruded, the fibers are submitted to a cross-linking treatment. For example, the fibers may be submitted to one or more of UV cross-linking, temperature-assisted cross-linking, or cross-linking induced by a cross-linking agent (e.g., enzymatic, ionic or other chemical cross-linking agent), as described in further detail below.

Once the fibers are cross-linked, the composition coating the biocompatible hydrogel is removed (as the biocompatible hydrogel is cross-linked and therefore maintains it structure by itself, and thus no longer requires the structural support of the coating). In some examples, the non-toxic polymer composition is a thermoreversible gelation non-toxic polymer which transitions from sol into a gel state. That is, the composition exhibits solid properties at a first temperature whilst being extrudable, such that the composition may be used as the printable composition 140 discussed above, and at a second temperature the composition may behave as a Newtonian fluid such that the composition may be removed by changing the temperatures of the extruded fibers. For instance, the composition 140 is removed by washing or incubating the printed fibers with a solution at a temperature which induces gel to sol state change of the thermoreversible polymer. For example, Poloxamer 407 behaves as a Newtonian liquid at low temperature (e.g. at 4 °C), and undergoes a sol-gel transition at room temperature (20 °C to 25 °C), showing a strong shear-thinning behaviour making it printable. It is preferable that the composition is solid at a temperature between 20 °C to 25 °C. Such compositions may be removable by a wash with a cold (e.g. a temperature between 0 °C and 4 °C, preferably about 4°C) aqueous solution, such as water or phosphate buffered saline applied to the extruded biological fibers.

**In** some embodiments, the steps of submitting the fibers to a cross-linking treatment may be performed at the same time as removing the composition coating the extruded fiber. For example, in embodiments where a solution containing a cross-linking agent (for example an ionic, enzymatic or other chemical cross-linking agent) is applied, the solution may be applied to the fibers at a temperature which causes the polymer composition to dissolve. For instance, in embodiments where Poloxamer 407 is used as the composition 140, the fibers may be washed with a solution containing cross-linking agent which has a temperature of about 4 °C, such that the solution both washes the composition 140 from the fibers, and also cross-links the fibers.

**In** other embodiments, the polymer composition 140 may comprise a cross-linking agent such that the cross-linking treatment is applied to the extruded biological fibers (with the polymer composition 140 further providing structural support to the biological fibers). The combination of physical and chemical confinement provides for a finer control of the fibers width.

As described above, the fibers may be submitted to any cross-linking method, for instance one or more of UV cross-linking, temperature-assisted cross-linking, or cross-linking induced by a cross-linking agent (e.g., enzymatic, ionic or other chemical cross-linking agent). Thus, the bioprinting system and method described herein is universal since it does not limit the nature of the biocompatible hydrogel.

For example, in some embodiments, the biocompatible hydrogel 130 may comprise a substance which crosslinks upon exposure to a cross-linking agent, wherein the extruded biological fiber is cross-linked exposing the obtained fiber to the cross-linking agent. As noted previously, the polymer composition 140 may comprise the cross-linking agent such that cross-linking occurs simultaneously to extrusion; in other examples, the biological fibers may be extruded, and the cross-linking agent performed in a separate step (for example after the polymer composition 140 is removed). In some embodiments the biocompatible hydrogel 130 comprises one or more of alginate, modified alginate comprising inserted cell attachment sites (e.g., Arg-Gly-Asp (RGD) motifs), such as in Alginate-RGD bioink (Sigma Aldrich), gellan gum and chitosan. Any of these components, or a combination thereof, may be at a concentration from 0.25% to 2% (wt/v), such as 0.5%, 0.75%, 1%, 1.25%, 1.5% or 1.75% (wt/v). In other embodiments, the biocompatible hydrogel 130 does not comprise alginate. In additional embodiments, it does not comprise alginate, modified alginate comprising inserted cell attachment sites (e.g., Arg-Gly-Asp (RGD) motifs), such as in Alginate-RGD bioink (Sigma Aldrich), gellan gum nor chitosan.

Where the biocompatible hydrogel comprises alginate, modified alginate comprising inserted cell attachment sites or gellan gum, the cross-linking agent may comprise a divalent cation, like Ca²⁺, Ba²⁺, Mg²⁺ and can be found at a concentration of 10 - 300 mM, preferably of 25 - 300 mM. In preferred embodiments, the ionic cross-linking agent comprising a divalent cation is CaCl₂. In other preferred embodiments, the polymer composition 140 does not comprise a divalent cation.

Where the biocompatible hydrogel comprises chitosan, the cross-linking agent may comprise negatively charged ions, preferably wherein the cross-linking agent comprises negatively charged Molybdenum or Platinum ions. For example, the biocompatible hydrogel 130 may comprise alginate having a concentration of 0.5% to 2% (wt/v). The polymer composition may comprise CaCl₂ at a concentration of 10 mM to 300 mM, preferably of 25 - 300 mM. In other embodiments the biocompatible hydrogel 130 may comprise one or more of the biocompatible polymers described herein above, preferably selected from collagen, gelatin, chitosan or combinations thereof and said cross-linking agent is another chemical cross-linking agent, including but not limited to genipin, proanthocyanidin and epigallocatechin gallate (Pinheiro A, Cooley A, Liao J, Prabhu R, Elder S. Comparison of natural crosslinking agents for the stabilization of xenogenic articular cartilage. J Orthop Res. 2016;34(6):1037-1046. doi:10.1002/jor.23121). As the polymer composition contains the cross-linking agent, the amount of cross-linking agent which is extruded is highly controllable as opposed to the use of cross-linking liquid phase solution, which is hard to control and can cause blockage of the printing system. In other embodiments, the polymer composition 140 does not comprise negatively charged ions.

In other embodiments, the biocompatible hydrogel 130 may comprise a substance which crosslinks upon exposure to UV light, and the crosslinking treatment may include exposing the extruded biological fiber to UV light. Examples of such substance include any hydrogel modified with acrylamide groups (i.e., a poly(acrylic) acid hydrogel), such as gelatin methacrylate (GelMA), poly(ethylene glycol) diacrylate (PEGDA), alginate methacrylate (AlgMA), collagen methacrylate (ColMA) or hyaluronan methacrylate (HA-MA). In other embodiments, the polymer composition 140 does not comprise acrylate polymers, such as described herein, or another substance which crosslinks upon exposure to UV light.

In some embodiments, the biocompatible hydrogel 130 may comprise a substance which crosslinks upon heating, and the crosslinking treatment may comprise heating the biological fiber, generally to a temperature of more than 30 °C, preferably about 37 °C during at least 30 minutes, preferably 1 hour. For example, the biocompatible hydrogel 130 may comprise one or more of collagen, a decellularized extracellular matrix (ECM) or other cell matrices, such as Matrigel^{®}. For example, the biocompatible hydrogel may comprise collagen at a concentration of about 2 mg/mL to about 10 mg/mL. In another example, the biocompatible hydrogel may comprise a decellularized extracellular matrix (ECM) or other cell matrices, such as Matrigel^{®} at concentrations of between 25% and 75% (v/v). It will be appreciated that other combinations of substances may be used for the biocompatible hydrogel, so long as the hydrogel is extrudable and has a high enough viscosity such that it does not diffuse into the outer polymer coating during cross-linking.

In some embodiments, the biocompatible hydrogel 130 may comprise a substance which crosslinks enzymatically, and the crosslinking treatment may comprise applying an enzymatic cross-linking agent to the biological fiber. The agent may be comprised in the polymer composition 140 such that the steps of extruding the fiber and performing the cross-linking treatment occurs simultaneously. **In** other embodiments, the cross-linking agent may be separately applied to the biological fiber. For example, the biocompatible hydrogel 130 may comprise fibrinogen and the enzymatic cross-linking agent may be an enzyme solution comprising thrombin, for example at a concentration of 5 U/mL to 20 U/mL. **In** other examples, the biocompatible hydrogel may comprise fibrinogen or gelatin and the enzymatic cross-linking agent may be an enzyme solution comprising transglutaminase. Enzymatic cross-linking is typically conducted at room temperature.

Of course, it will be appreciated that in some embodiments, any combination of the above examples of UV, temperature-assisted, or cross-linking-agent induced is used. For example, the biocompatible hydrogel 130 may comprise at least a first cross-linkable substance and a second cross-linkable substance, wherein the cross-linking of the second substance is reversible. The second cross-linkable substance may be removed from the biocompatible hydrogel after the cross-linking treatment is applied for the first and second cross-linkable substances. This may be preferably when the second cross-linkable substance is useful in providing an initial structure to the obtained fibers, but may inhibit cell proliferation and differentiation. For example, the second substance may be alginate and the alginate may be removed by application of a calcium chelator, such as ethylenediaminetetraacetic acid (EDTA), egtazic acid (EGTA), citric acid or etidronic acid. **In** preferred embodiments, said calcium chelator is EDTA which can be used for example at a concentration of 10mM-30mM, preferably about 20mM.

It will be appreciated by the skilled person that a variety of substances may be used in both the biocompatible hydrogel 130 and the polymer composition 140. The physical confinement of the biocompatible hydrogel 130 by the polymer composition 140 relies on the gelation of the hydrogel 130 and polymer composition 140 such that diffusion of the biocompatible hydrogel 130 through the polymer composition 140 is negligible. It will be appreciated that for any given composition, the optimal concentrations for gelation can be determined by routine experimentation. **In** cases where the polymer composition additionally comprises a cross-linking agent such that cross-linking occurs immediately upon extrusion, this may immediately chemically confine the biocompatible hydrogel 130 inside the polymer composition such that the gelation of the biocompatible hydrogel 130 and the polymer composition 140 may take a wider range of values (as the chemical cross-linking inhibits diffusion of the biocompatible hydrogel).

It is also noted that higher gelation may reduce the rate of extrusion for a given pressure, which may provide an upper limit for the concentrations, depending on the pressure that may be applied by the printing system.

It will be appreciated that many combinations of biocompatible hydrogels and polymer compositions are possible. The fiber quality is influenced by both the homogeneity of the printing and the stability of the fibers. For instance, physical confinement of a hydrogel only containing decellularized extracellular matrix (ECM) or other cell matrices, such as Matrigel^{®} or collagen might give homogeneous fibers if the concentration of gelatin is high enough, but their stability is low, as their stiffness of the construct after temperature crosslinking is low. Therefore, thin and homogeneous fibers can be printed with this combination, but they are more easily broken. Adding fibrinogen to the mixture increases their long-term stability, as fibrin is more robust, creating high quality fibers. In preferred embodiments, the biocompatible hydrogel 130 comprises fibrinogen, preferably comprises or the polymers in said biocompatible hydrogel consist of:
- fibrinogen and gelatin;
- fibrinogen, gelatin and a protein matrix or ECM-hydrogel, such as Matrigel^{®};
- fibrinogen, gelatin and hyaluronic acid.

Gelatin may be at a concentration from 1% to 6%, preferably from 3% to 5%, such as 3.5%. Fibrinogen may be at a concentration from 10 mg/mL to 30 mg/mL, preferably about 20 mg/mL. The protein matrix or ECM-hydrogel may be at a concentration from 25% to 75% (v/v), preferably from 40% to 60% (v/v), such as about 50% v/v. In preferred embodiments, the concentration of these components is as described in Table 1 and in the examples.

A similar effect occurs when the polymer composition 140 contains a cross-linking agent. For example, the polymer composition may comprise CaCl₂ and the biocompatible hydrogel may comprise alginate. If the main biomaterial is too liquid, like Matrigel^{®}, it will diffuse through the polymer composition before the Ca-crosslinking of alginate can form homogeneous fibers, unless the concentration of alginate is increased to accelerate this process. Since alginate does not have cell attachment motives, it is preferable to keep its concentration as low as possible; in that case, however, the quality of the fibre will not be high.

One of the best strategies to follow is the combination of both methods by adding a small amount of gelatin (e.g., from 1% to 6%, preferably from 3% to 5%, such as 3.5%) or GelMA (e.g., from 2% to 10%, preferably from 5% to 7%, such as 6%) to alginate, using a hybrid chemical-physical method of confinement, wherein the biocompatible hydrogel is physically confined by its gelation properties, and is further cross-linking immediately upon extrusion. With this combination, as it can be seen in Table 1, the quality of the fibers is much improved and virtually any material can be used with it.

Collagen and Matrigel^{®}, which are crosslinked slowly at 37 °C during approximately 30 min in an irreversible manner, deserve special consideration. Collagen is one of the main components of many tissue's ECM and, in particular, of skeletal muscle tissue, making it especially interesting for 3D bioengineering applications. Also, Matrigel^{®} is one of the most widely used basement membrane matrices for 2D and 3D culture, since it is rich in collagen and many other ECM proteins, but shares the same difficulties. However, their irreversible and low temperature-dependent crosslinking makes their bioprinting difficult, as opposed to gelatin, which has reversible crosslinking. Both materials are liquid at room temperature and cannot be 3D-bioprinted by pneumatic extrusion, but if they are crosslinked at 37 °C, they are also too stiff to be extruded. Although the most promising approaches in the literature have dealt with the mixture of alginate and collagen to achieve proper extrusion, better strategies are needed in the field. One of the main difficulties with these approaches is due to the slow crosslinking of these materials. Because of this, the printed construct can easily lose its shape before the crosslinking has taken place. For that reason, collagen or Matrigel^{®} have been mainly used with casting molds, which can retain the shape of the construct until the biocompatible hydrogel is fully crosslinked. With the printing systems and methods disclosed herein, however, these materials can be protected by the coating of the polymer composition 140 during crosslinking at 37 °C, avoiding their diffusion in the media. Using a polymer composition that does not dissolve at high temperatures, such as the poloxamer 407, fibres can be incubated in physiological temperatures for 30-45 minutes until Matrigel^{®} or collagen have been crosslinked, and then removed with cold PBS. Any confinement scheme (either chemical or physical) could potentially be used in combination with this type of biocompatible hydrogel. Additionally, the process could include a tertiary crosslinking with fibrinogen to improve the mechanical stability of the fibres.

Table 1 shows a list of preferred examples. It will be appreciated that other combinations of cross-linking substances are possible, in which case other concentrations may be possible so long as the biocompatible hydrogel and the polymer compositions are extrudable, and the relative viscosity of the biocompatible hydrogel inhibits diffusion of the biocompatible hydrogel through the polymer composition.

**Table 1.- Relation of different strategies to obtain thin, independent fibres, combining different biomaterials, confinement strategies and crosslinking methods. * The fiber quality is influenced by both the homogeneity of the printing and the stability of the fibers (e.g. not breaking during extrusion, Poloxamer 407 removal or further manipulation).**

| **Biological hydrogel** | | **Type of crosslinking** | **Polymer composition** | | **Fiber quality*** |
|---|---|---|---|---|---|
| **Material** | **Concentration** | | **Material** | **Concentration** | |
| Collagen | 2 - 6 mg/mL | Temperature | poloxamer 407 | 33% - 40% (wt/v) | Acceptable |
| Gelatin | 3% - 5% (wt/v) | | | | |
| Matrigel^{®} | 25% - 75% (v/v) | Temperature | poloxamer 407 | 33% - 40% (wt/v) | Acceptable |
| Gelatin | 3% - 5% (wt/v) | | | | |
| Fibrinogen | 10 - 30 mg/mL | Enzymatic | poloxamer 407 | 33% - 40% (wt/v) | High |
| Gelatin | 3% - 5% (wt/v) | | | | |
| Fibrinogen | 10 - 30 mg/mL | Enzymatic Temperature | poloxamer 407 | 33% - 40% (wt/v) | High |
| Matrigel^{®} | 25% - 75% (v/v) | | | | |
| Gelatin | 3% - 5% (wt/v) | | | | |
| Matrigel^{®} | 25% - 75% (v/v) | Temperature Ionic | poloxamer 407 + CaCl₂ | 33% - 40% (wt/v) | Medium |
| Alginate | 0.25% - 2% (wt/v) | | | | |
| Matrigel^{®} | 25% - 75% (v/v) | Temperature Ionic | poloxamer 407 + CaCl₂ | 33% - 40% (wt/v) 25 - 300 mM | High |
| Alginate | 0.25% - 2% (wt/v) | | | | |
| Gelatin | 3% - 5% (wt/v) | | | | |
| Fibrinogen | 10 - 30 mg/mL | Enzymatic | poloxamer 407 + CaCl₂ | 33% - 40% (wt/v) | High |
| Gelatin | 3% - 5% (wt/v) | | | 25 - 300 mM | |
| Alginate | 0.25% - 2% (wt/v) | Ionic | | | |
| Fibrinogen | 10 - 30 mg/mL | Enzymatic Temperature | poloxamer 407 + CaCl₂ | 33% - 40% (wt/v) | High |
| Matrigel^{®} | 25% - 75% (v/v) | | | 25 - 300 mM | |
| Gelatin | 3% - 5% (wt/v) | | | | |
| Alginate | 0.25% - 2% (wt/v) | Ionic | | | |
| Fibrinogen | 10 - 30 mg/mL | Enzymatic | poloxamer 407 + CaCl₂ | 33% - 40% (wt/v) | High |
| GelMA | 4.5% - 7 % (wt/v) | UV | | 25 - 300 mM | |
| Alginate | 0.25% - 2% (wt/v) | Ionic | | | |
| Fibrinogen | 10 - 30 mg/mL | Enzymatic Temperature | poloxamer 407 + CaCl₂ | 33% - 40% (wt/v) | High |
| Matrigel^{®} | 25% - 75% (v/v) | | | 25 - 300 mM | |
| GelMA | 4.5% - 7 % (wt/v) | UV | | | |
| Alginate | 0.25% - 2% (wt/v) | Ionic | | | |

Table 1 presents a guide to understand the possible combinations of materials according to their confinement method. It also provides illustrative concentrations of the biocompatible hydrogel materials, as well as of the polymer composition and even CaCl₂.

The fibers as described herein are preferably non-hollow fibers. In particular embodiments, the biocompatible hydrogel 130 does not comprise poloxamer 407 (i.e., Pluronic^{®} F-127).

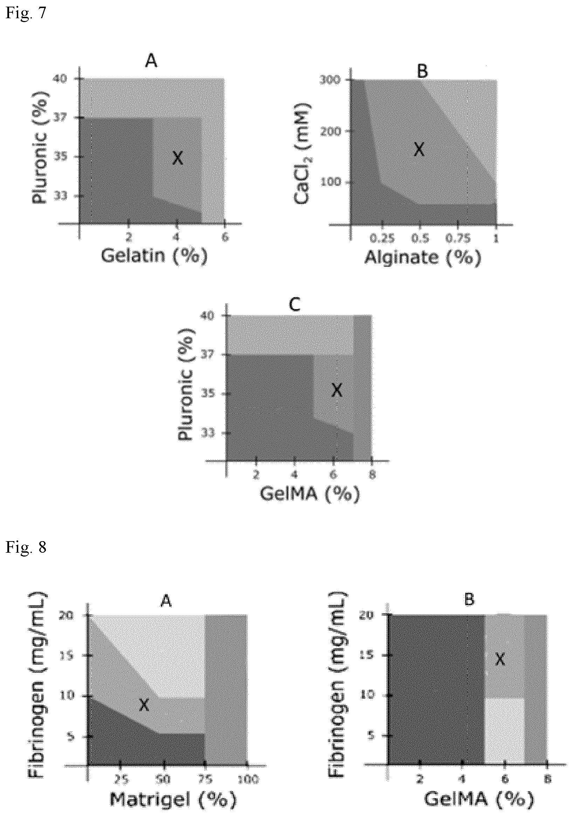

FIG. 7 focuses on the description of these possibilities and shows a number of graphs illustrating possible combination of materials in the biocompatible hydrogel and the non-toxic polymer to achieve a homogeneous individual fiber.

Graph A illustrates optimal concentrations of poloxamer 407 (Pluronic^{®} F-127) as the polymer composition, without a cross-linking agent, combined with gelatin in the biocompatible hydrogel. The concentration of gelatin with respect to the concentration of poloxamer 407 needs to be adjusted to avoid diffusion of the biocompatible hydrogel through the pluronic. In general, gelatin in concentrations below 3% (wt/v) diffuses, since its gelation is not high enough. At the same time, pluronic at concentrations below 33% (wt/v) causes the same problem. The preferred range of concentrations for gelatin to get homogeneous fibers, indicated by the region marked "X", is between 3-5% (wt/v); for the poloxamer 407, it is between 33-37 % (wt/v). Higher concentrations of both pluronic and gelatin can still be used successfully; however, they might require pressures too high for the 3D bioprinter (this depends on the specific printing system) and cells might suffer too much shear stress during extrusion.

The chemically assisted physical confinement method, based on alginate crosslinking on extrusion (e.g. induced by CaCl₂), provides the best homogeneity of fibers compared to physical confinement based on relative gelation properties. Moreover, it does not present clogging problems, since the flow of the polymer composition can be carefully controlled by the applied pressure. Whilst alginate also depends on the concentration of pluronic in the same way as gelatin, it is more dependent on the concentration of CaCl₂ dissolved in it with respect to its own concentration. Alginate concentrations ranging from as low as 0.25% (wt/v) can yield homogeneous fibers if the concentration of CaCl₂ is high enough and other materials that increase the viscosity are present in the mixture. Higher concentrations of alginate, even surpassing 1% (wt/v) can, naturally, produce very homogeneous fibers, but its stiffness might be too high for the cells, taking into account that other biocompatible polymers with attachment sites, like fibrinogen or collagen, may be included in the biocompatible hydrogel. If the presence of alginate is a problem for cell proliferation or differentiation due to low biocompatibility, alginate can be removed after crosslinking of the other cross-linkable materials in the biocompatible hydrogel by the addition of ethylenediaminetetraacetic acid (EDTA) for 5 min. EDTA is a calcium chelator that will attract the Ca+2 ions that reversibly crosslink alginate, making it dissolve in the culture medium. In this case, it would be advisable to keep the concentration of alginate low, for instance around 0.5 % (wt/v), with CaCl₂ molarities between 100-200 mM, in order to make it easier to remove. Graph B illustrates optimal concentrations of CaCl₂ in a polymer composition, combined with alginate in a biocompatible hydrogel which additionally comprises gelatin at 3% (wt/v) as extra support. The preferred range of concentrations for obtaining homogeneous fibers is indicated by the region marked "X". If the concentration of alginate and CaCl₂ is too high, then the fibers are too stiff. Conversely, if the concentration is too low then the fibers do not form.

Similar to graph A, graph C illustrates the optimal concentrations of poloxamer 407 (Pluronic) as the polymer composition, without a cross-linking agent, combined with GelMA rather than gelatin in the biocompatible hydrogel. The preferred range of concentrations for obtaining homogeneous fibers is indicated by the region marked "X". It is noted that GelMA gelifies at higher concentrations than gelatin, and a minimum concentration of 5% (wt/v) should be used, compared to the minimum of 3% (wt/v) for gelatin. This creates a smaller range to obtain homogeneous fibers, between 5-7% (wt/v). For concentrations higher than this, the mixture is mostly unextrudable. Nevertheless, homogeneous fibers can be obtained with this method by choosing the concentrations as shown in the region marked "X" in graph C. Further, alginate alginate may additionally be used to provide extra support. If GelMA is later on crosslinked with UV light, the material will remain in the biocompatible hydrogel, providing cell attachment sites, instead of being washed away, as in the case of gelatin. Further optimization may be achieved by mixing both gelatin and GelMA, to regulate density of GelMA that will remain in the mixture after UV crosslinking.

FIG. 8 shows a number of graphs illustrating possible combinations of materials that provide sufficient cell attachment sites. Graph A illustrates preferred concentrations of fibrinogen and Matrigel^{®} in the biocompatible hydrogel. When both fibrinogen and Matrigel^{®} are used, the resulting matrix after crosslinking might be too dense for cell proliferation. For instance, if Matrigel^{®} is mixed at a 50% (v/v) concentration, fibrinogen should be decreased to the range of 5 mg/mL-15 mg/mL, preferably around 10 mg/mL. However, if fibrinogen is reduced too much, the density of attachment sites of Matrigel^{®}, as well as its stiffness, is not high enough for proper cell differentiation. The preferred range of concentrations is indicated by the region marked "X".

Graph B illustrates preferred concentrations of fibrinogen and Matrigel^{®} in the biocompatible hydrogel. If GelMA is used as a confinement material, instead of gelatin, which is dissolved during incubation, its proportionality with respect to fibrinogen is also important. As already mentioned, GelMA concentrations below 5% (wt/v) do not produce homogeneous fibers, since they dissolve through the pluronic. In this range of applicability, fibrinogen should be kept between 10-20 mg/mL to have enough attachment sites. The preferred range of concentrations is indicated by the region marked "X".

If fibrinogen is used alone as the main component for the biocompatible hydrogel (e.g. for a myoblast-laden hydrogel), it should be kept at a high concentration, with 20 mg/mL being a preferred value for cell differentiation.

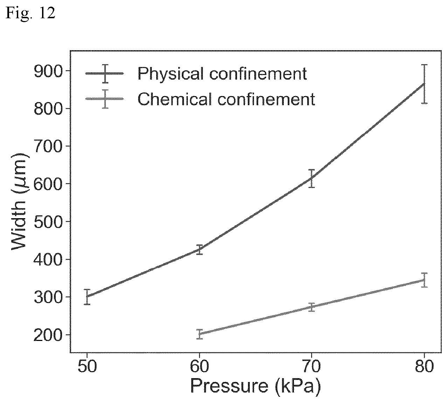

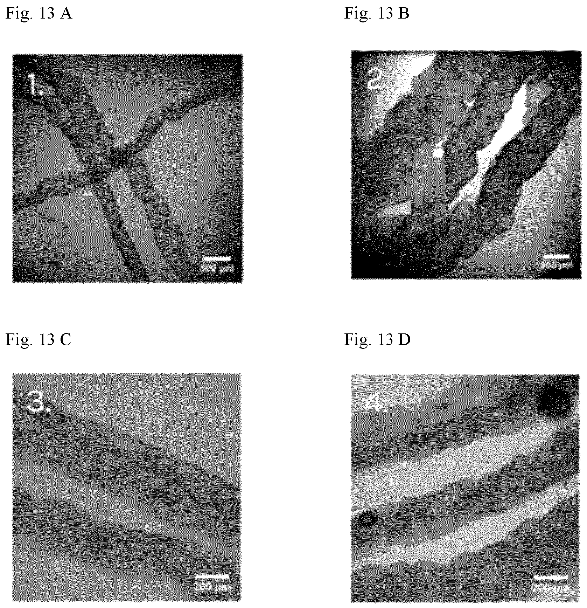

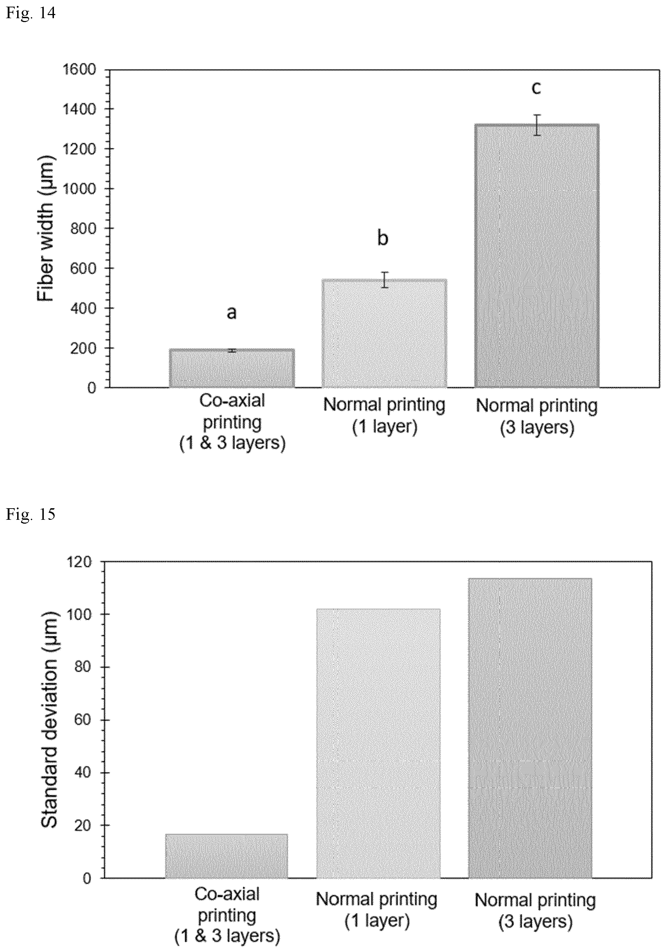

In examples where the biocompatible hydrogel is confined due to the gelation properties of the polymer composition 140 alone (i.e. without a cross-linking agent in the polymer composition 140), it has been observed that for an inner nozzle diameter of 200 µm, biological fibers having a diameter (e.g. mean diameter) of between about 200 µm and about 900 µm, such as between about 300 µm and about 900 µm are obtainable by varying the pressure applied to the inner nozzle between 40 kPa and 80 kPa. In an example where a cross-linking agent is used in the polymer composition such that in additional to the physical confinement, cross-linking occurs simultaneously to extrusion, it has been observed that for an inner nozzle diameter of 200 µm, biological fibers having a diameter (e.g. mean diameter) of between about 200 µm and about 300 µm are obtainable by varying the pressure applied to the inner nozzle between 60 kPa and 80 kPa. FIG. 12 shows a graph illustrating the width of the homogeneous fibers obtained by physical confinement of the fibers (i.e. due to the gelation properties alone) and by chemically assisted physical confinement (using simultaneous cross-linking during extrusion), with an inner nozzle diameter of 200 µm. It is noted that fibers with lower and higher thickness than those shown in FIG. 12 can be obtained by varying the pressure further. If the applied pressure is too low then the fibers may not be continuous. Furthermore, it is noted that if the diameter of the inner nozzle is changed, then a different range of fiber diameters will be obtainable by varying the pressure. FIGs. 13A to 13D illustrate images of homogeneous fibers obtained at points 1 to 4 on the graph shown in FIG. 12. As can be seen, the fibers obtained have a high homogeneity. In another example it has been observed that for an inner nozzle diameter of 200 µm, biological fibers having a diameter (e.g. mean diameter) of about 200 µm are obtainable by the physical confinement method of the invention (see FIG.14).

In some embodiments, the barrel of the internal (first) nozzle is at a pressure from 30 to 120 kPa, preferably from 40 to 80 kPa. By changing the relative pressure applied to the nozzles, the diameter of the extruded biological fiber can be varied relative to the diameter of the first nozzle 110. For instance, the barrel of the external (second) nozzle is at a pressure from 250 to 350 kPa and the barrel of the internal (first) nozzle at a pressure from 30 to 120 kPa, preferably from 40 to 80 kPa. In a preferred embodiment, the first nozzle has a diameter of 200 µm and the second nozzle has a diameter of 800 µm; and the barrel of the external (second) nozzle is kept at a pressure from 250 to 350 kPa and the barrel of the internal (first) nozzle at a pressure from 40 to 80 kPa.

The printing systems and methods disclosed herein may be used for tissue engineering purposes. In a particular embodiment, one or more obtained individual fibers may be comprised in or form a biomimetic structure (e.g. a tissue construct). Said biomimetic structure can further comprise a biomaterial. The term "biomaterial" may refer to any biocompatible material which serves as a substrate or guide for tissue repair or regeneration, for instance tissue scaffolds, tissue implants, stents or valves.

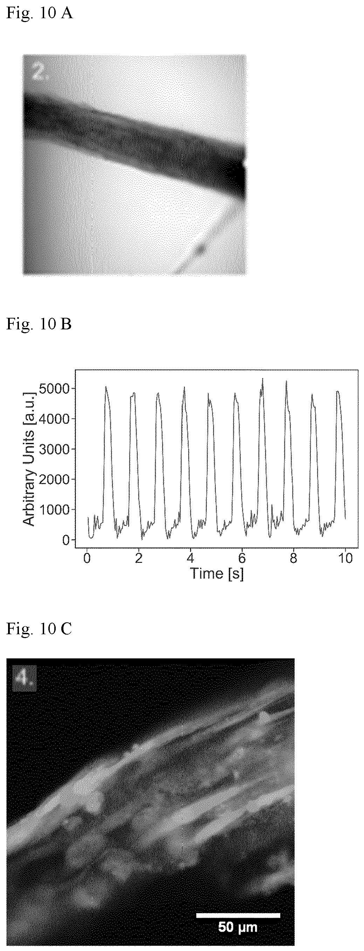

In some embodiments, the 3D-printed fibers of the invention can be used for the 3D bioengineering of tissue and obtaining of tissue constructs. These tissue constructs may be embedded with or without cells. These cells may be as described herein above. In preferred embodiments, said tissue is skeletal muscle tissue, preferably human or mouse skeletal muscle tissue. As shown in the Examples, the inventors have obtained a skeletal muscle construct with the 3D printing method and system of the invention with aligned myotubes and good contractibility features (see Fig.10).

The obtained biomimetic structure (e.g. tissue construct) can be used for research purposes. For instance, the obtained tissue can be used for drug testing purposes. The obtained biomimetic structure (e.g. tissue construct) can also be used for medical purposes, such as for tissue replacement or regeneration purposes.

In a particular embodiment of any of the above, the individual fibers obtained by the printing system and methods of the invention comprise skeletal muscle myotubes. As described above, myoblasts may be embedded in the hydrogel 130 and myoblasts may be induced to differentiate into multi-nucleated myotube structures.

Using the printing systems and methods disclosed herein, multiple individual fibers (such as that shown in FIG.2A) may be obtained which are aligned in the same uniaxial direction(either with or without the polymer coating, depending on if it is removed after cross-linking).

Biological actuators based on 3D-printed skeletal muscle tissue can be used to study muscle development, maturation or healing, or even act as drug testing platforms for muscle; and more complex, untethered actuators can take the form of hybrid biocompatible machines comprising 3D-printed muscle fibers. Such hybrid biocompatible machines may comprise one or more individual fibers obtained by a bioprinting system as disclosed herein.

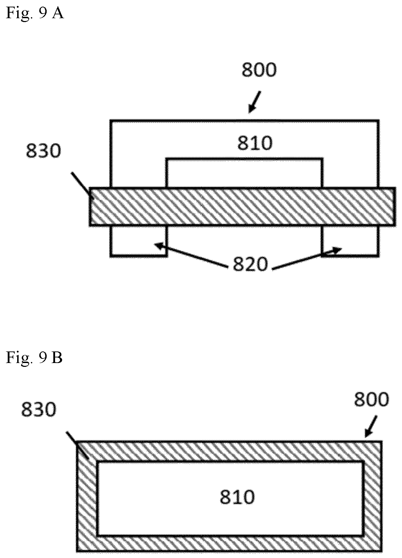

For example, FIGs. 9A and 9B show a hybrid biocompatible machine 800 according to one or more embodiments shown and described herein. The machine 800 comprises a main body 810 and two legs 820 extending from the main body 810. The main body is formed of a flexible material such as Polydimethylsiloxane (PDMS) or Poly(ethylene glycol) diacrylate (PEGDA). One or more individual biological fibers 830 (e.g. comprising skeletal muscle myotubes) obtained by a printing system disclosed herein extend in a loop about the legs 820. When the biological fibers 830 are stimulated to contract, the contracting force is transmitted to the legs 820 of the machine 800 and the main body 810 is caused to flex.

The machine 800 could be provided with an asymmetry such that contraction of the one of more biological fibers 830 causes the machine 800 to translate in a particular direction.

In other embodiments, the individual fibers printed using the printing systems and methods disclosed herein could be used in other biological machines, such as tethered biological actuators, for example as disclosed in *Force Modulation and Adaptability of 3D-Bioprinted Biological Actuators Based on Skeletal Muscle Tissue* (Rafael Mestre et al.; Adv. Mater. Technol. 2019, 4, 1800631).

The hybrid biocompatible machines described above can be used for a variety of purposes, such as evaluation of tissue differentiation, functionality, drug testing platforms, drug screening platforms, force measurement platforms or as tissue models of young or old muscle (for example by assessing morphological and functional changes in the aging process of muscular tissue). In particular, the hybrid biocompatible machine may be studied for the force generation and contraction profiles of the printed fiber, allowing for a more detailed analysis of the tissue.

### EXAMPLES

### Material & Methods

The assays disclosed below were carried out using the following materials and methods:

### Fabrication of inner and outer nozzles

The co-axial nozzles were manually fabricated using different types of commercially available nozzles and tips. The inner nozzle, where the cell-laden hydrogel passed through, was a 200µm (G27) plastic conical nozzle (Optimum^{®} SmoothFlow^{™} tapered tips, Nordson^{®}, ref. 7018417). The luer lock of this nozzle was left free to be connected to the first bioprinting barrel, where the cell-laden hydrogel would be loaded. The outer nozzle that covered the inner one was a filtered P1000 pipette tip (Labclinics, ref. LAB1000ULFNL), cut approximately at 5 cm from its end. The tip was trimmed to increase its diameter at the final point. Both nozzles were assembled and glued together. When the glue was dry and the assembly stable, a hot puncher was used to create a hole in the outer nozzle, at approximately 1.5 cm from its ending, with care to not create another whole in the internal nozzle. The secondary nozzle, where poloxamer 407 flowed, was inserted inside this hole. This secondary nozzle was a flexible polypropylene 800µm nozzle (G18) from Nordson^{®} (EFD^{®} 7018138). A silicone tubing of 0.8 mm of diameter (ibidi, ref. 10841) was attached to this external nozzle through a male elbow luer connector (ibidi, ref. 10802). A 1.1-mm (19G) nozzle (BBraun Sterican^{®}, ref. 4657799) was inserted through the other end of the tubing, so that its luer lock connector could be connected to the second bioprinting barrel, containing poloxamer 407 acid.

### Fabrication of hydrogel and polymer composition

For the fabrication of the different combinations of hydrogels, the following materials were used: gelatin from porcine skin, type A (Sigma-Aldrich, G2500), fibrinogen from bovine plasma (Sigma-Aldrich, F8630) with thrombin from bovine plasma (Sigma-Aldrich, T4648) as crosslinker, Matrigel^{®} Basement^{™} membrane matrix (Corning^{®}, 354234), sodium alginate (Sigma-Aldrich, W201502), GelMA with lithium phenyl-2,4,6-trimethylbenzoylphosphinate (LAP) at 0.25% (wt/v) as photoinitiator (CELLINK^{®}, LIK-3050V-1), collagen type I high concentration (Corning^{®}, 354249), and Pluronic^{®} F-127 powder (Sigma-Aldrich, P2443).

Poloxamer 407 was dissolved at concentrations ranging from 30-40% (wt/v) in ultrapure water, further comprising CaCl2 (at molar concentrations ranging from 50 mM to 300 mM) for those fibers obtained by the chemically-assisted physical confinement method, under stirring in a refrigerator (4 °C) until fully dissolved. For hydrogels containing gelatin, alginate and/or GelMA in the same composition, these components were mixed together in PBS at the desired concentrations. If the hydrogel contained fibrinogen, this component was dissolved in PBS at a double concentration and then added to the hydrogel containing alginate and/or gelatin, also at a double concentration, in order to avoid pipetting the viscous mixture. If the hydrogel also contained Matrigel^{®}, the concentrations were also adjusted to achieve the desired concentrations reducing the need for pipetting (for instance, at 1:1:1 ratios, or 2:1:1 ratios, etc.).

### 3D bioprinting

C2C12 myoblasts were harvested by a 0.25% (wt/v) Trypsin-0.53 mM EDTA solution, centrifuged at 300g for 5 min and the pellet re-suspended at a concentration of 5x10⁶ cells/mL in the hydrogel mixture, at 37 °C. The cell-laden hydrogel was loaded into a 3-mL plastic syringe (Nordson^{®}, ref. 7012085) coupled to the inner nozzle of the co-axial nozzle.

Poloxamer 407 was loaded while cold into a secondary barrel and left at RT to gellify beforehand. CELLINK^{®} Inkredible+ 3D bioprinter (CELLINK^{®}, Sweeden) was used to bioprint the hydrogel fibers. The cell-laden hydrogel was inserted in the first cartridge and the poloxamer 407 barrel to the second barrel, and all the nozzles connected as previously explained. The pressure for the pluronic barrel was kept between 250-350 kPa and adjusted manually, although these values are highly dependent on the diameter and length of the silicone tubing and the concentration of pluronic. For the hydrogel, the pressures were kept between 40-80 kPa, also adjusted manually, depending on the type of hydrogel. The designs were directly written in GCode with the help of the open-source software Slic3r (v. 1.2.9) and the bioprinter was controlled with RepetierHost (v. 2.0.5).

After 3D bioprinting, if the hydrogel contained fibrinogen, a solution of 5 U/mL of thrombin was added to the Petri dish for 5 min in a refrigerator at 4 °C. At this point, poloxamer 407 would dissolve and fibrinogen would crosslink to form fibrin. After this, several washes with cold PBS were done to completely remove poloxamer 407, If the hydrogel contained Matrigel^{®}, the as-printed constructs were left in an incubator for at least 30 min, without removing pluronic. After this, a cold solution of PBS (or thrombin, if fibrinogen was also in the mixture) was added to dissolve pluronic after several washes. If the chemically assisted physical confinement method, based on the cross-linking of alginate by divalent cations, was used, a cold solution of thrombin was added to induce fibrinogen crosslinking and remove poloxamer 407; subsequently a solution of 20 mM EDTA (Sigma-Aldrich, E6758) adjusted with NaOH to pH 7, was added to the Petri dish after crosslinking, for 5 minutes for alginate removal. In all the cell laden hydrogels, Growth Medium supplemented with 6-aminocaproic acid (ACA) was added and the constructs were left in a cell incubator at 37 °C and 5% CO₂ atmosphere.

### Rheological characterization of poloxamer 407 acid

Rheological characterization of the Pluronic^{®} F-127 composition at the tested concentrations was performed using a Discovery HR-2 controlled-stress rheometer (TA Instruments) equipped with a Peltier steel cone geometry of 40 mm of diameter, 26 µm of truncation, and an angle of 1.019°. The Peltier element was set to 4 °C and 25 °C to demonstrate the behaviour of the block co-polymer at low and room temperatures. In all experiments, the sample was left to acquire the desire temperature for 1 min. A flow ramp with shear rate from 100 1/s to 0.01 1/s was performed, in logarithmic mode with 600 s of duration per point, with a pre-conditioning to the temperature of 30 s and pre-shear of 3 rad/s for 10 s.

### Cell culture, differentiation and electrical stimulation

C2C12 mouse myoblasts were purchased from ATCC and maintained in growth medium (GM) consisting of high glucose Dullbecco's Modified Eagle's Medium (DMEM; Gibco^{®}) supplemented with 10% Fetal Bovine Serum (FBS), 200 nM L-Glutamine and 1% Penicillin/Streptomycin, in a 37 °C and 5% CO2 atmosphere. Cells were passaged before reaching 80% confluency in Corning^{®} T-75 flasks.

For cell differentiation and myotube formation after the bioprinting process, GM was substituted by DM, consisting of high glucose DMEM containing 10% Horse Serum (Gibco^{®}), 200 nM L-Glutamine (Gibco^{®}), 1% Penicillin-Streptomycin (Gibco^{®}), 50 ng/ml IGF-1 (Sigma-Aldrich) and 1 mg/ml 6-aminocaproic acid (ACA, Sigma-Aldrich).

3D-bioprinted fibres were stimulated with a set of carbon-made electrodes attached to the cover of a Petri dish under an inverted microscope (Leica's DMi8) with pulses of 2 ms and 1 V/mm. Analysis of the contractions was performed with a home-made Python algorithm based on computer vision techniques that computed the distance between frames of a selected ROI by applying an L2-norm to the image pixels.

### Cell viability and Myosin Heavy Chain II (MHCII) immunofluorescence

Cell viability was analysed by the dual-fluorescence Live/Dead^{®} Viability/Cytotoxicity kit for mammalian cells (LifeTechnologies), following the manufacturer's instructions. Fluorescently labelled fibres were imaged under a Leica DMi8 inverted fluorescence microscope equipped with a 37 °C and 5% CO2 chamber, using a 20× air objective. The percentages of live and dead cells were calculated by using ImageJ software ver.1.47q (National Institutes of Health, Bethesda, MD).

For immunostaining, 3D-bioprinted constructs were washed twice in PBS and fixed by incubating them with a 3.7% paraformaldehyde in PBS solution for 15 min at RT, followed by three washes in PBS. Then, cells were permeabilized by using 0.2% Triton-X-100 in PBS. After two washes in PBS, the constructs were incubated with 5% Bovine Serum Albumin (BSA) in PBS (PBS-BSA) to block unspecific bindings. Then, bioprinted structures were incubated for 2 hours at RT and dark conditions with a 1/400 dilution of Alexa Fluor^{®}488-conjugated Anti-Myosin Heavy Chain II antibody (eBioscience) in 5% PBS-BSA. The unbound antibodies were washed out with PBS, and cell nuclei were counterstained with 1 µl/mL Hoechst 33342 (Life technologies). Finally, samples were washed twice in PBS and they were stored at 4 °C until their analysis. Fluorescently immunostained fibre constructs were imaged under a Zeiss LSM 800 confocal scanning laser microscope (CSLM), with a diode laser at 488 nm and 405 nm excitation wavelength for Myosin Heavy Chain II and cell nuclei.

### Force measurement assay

For force measurement, the tissue was printed around two 3D printed posts made of PDMS. The 2-post system (3 mm high, 0.5 mm wide and with 2 mm of lateral width) was 3D-printed beforehand with PDMS of a 1:20 and crosslinked at 37 °C for several days. Once cell-laden construct was bioprinted, the culture medium was changed to DM supplemented with ACA and IGF-1 (as defined in Example 1). To measure the fabricated tissue's final force, the protocol described by Mestre et al. (R. Mestre, T. Patiño, X. Barceló, S. Anand, A. Pérez-jiménez, S. Sánchez, Adv. Mater. Technol. 2018, 1800631) for force measurement in a two post-system for muscle-based actuators was used. The recording of the whole setup undergoing EPS was carried out inside an inverted microscope (DMi8, Leica), in a chamber that allowed to mimic physiological conditions (37 °C and 5% CO2). Their force measured by deflection of the posts by the cell-laden scaffold. Pulses of different frequencies of 1 ms were applied, keeping a constant voltage of 15 V. Euler- Bernoulli's beam bending equation was used to estimate the forces and stresses exerted against the posts to the tissue during electrical stimulation.

### Fiber width measurement

Bright-field images of bioprinted fibers were measured by drawing a line with the "measure" tool of ImageJ software ver.1.47q (National Institutes of Health, Bethesda, MD). A range of 5-12 fibers were measured for each case, as specified.

### EXAMPLE 1. Obtaining free-form width-controlled individual fibres with physical confinement and chemically assisted physical confinement methods of the invention

### EXAMPLE 1. 1 - Obtaining thin fibres with aligned myotubes from myoblast-cell laden fibers obtained by the chemically assisted physical confinement method

In a first example, a chemical confinement assisted strategy was carried out with C2C12 myoblast concentration of 5 million/mL and a hydrogel composed of 0.5% (wt/v) of alginate, 3% (wt/v) gelatin to increase its viscosity and help with the homogeneity of the fibers, and 20 mg/mL fibrinogen for cell attachment sites. For the polymer composition, poloxamer 407 at 33% (wt/v) with 300 mM CaCl2 was used as support material.

Upon extrusion at room temperature, Ca₊₂ ions in the external polymer composition entered in contact with alginate at the borders of the inner hydrogel, chemically cross-linking it to achieve a homogenous fiber. After 3D bioprinting using a printing system disclosed herein, a cold solution of thrombin (5 U/mL) was added to the construct for 5 min at 4°C. At this temperature, poloxamer 407 dissolved while fibrinogen was being crosslinked into fibrin. After this time, several washes with cold PBS removed completely the remaining poloxamer 407.

The use of a confinement method based on alginate, although it produces very homogeneous fibers, may be an acceptable environment for some cell types. However, it does not provide the optimal environment for myoblast differentiation. 3D-bioengineered skeletal muscle tissue generally shows passive compaction during the differentiation process. Alginate is a polysaccharide which does not possess cell attachment motifs. Therefore, individual fibers crosslinked with alginate, even at low a concentration of 0.5% (wt/v), did not show much compaction, something necessary for the proper differentiation of the cells. Crosslinking of alginate methacrylate (AlgMA) to a main network formed out of GelMA, for instance, has been proven to provide a suitable environment for this tissue. Likewise, chemically modifying alginate to insert cell attachment sites (such as RGD sequences) would also help in the compaction issue while taking advantage of this crosslinking method. This problem, nonetheless, could be solved without chemical modifications by the dissolution of alginate after the secondary crosslinking took place (and its presence was not necessary anymore) by the addition of a chelating agent, such as EDTA, for 5 min. EDTA can sequester calcium ions involved in the irreversible ionic crosslinking of alginate, therefore rendering it soluble. This strategy has been previously proven to remove most of the alginate present in the hydrogel without disturbing the attachment of cells (strongly affected by calcium chelators) in such a short time frame. In the experiment, 3D-printed fibers showed compaction after incubation with EDTA (see Fig. 10A), indicating the removal of alginate and the progression of the differentiation process. After 7 days, electric pulse stimulation (EPS) induced contractions in the fibers, as shown in Fig. 10B and immunostaining of Myosin Heavy Chain II and cell nuclei showed thin fibers with aligned myotubes (Fig. 10C).

### EXAMPLE 1.2.- Obtaining thin fibres with aligned myotubes from myoblast-cell laden fibers obtained by the physical confinement method

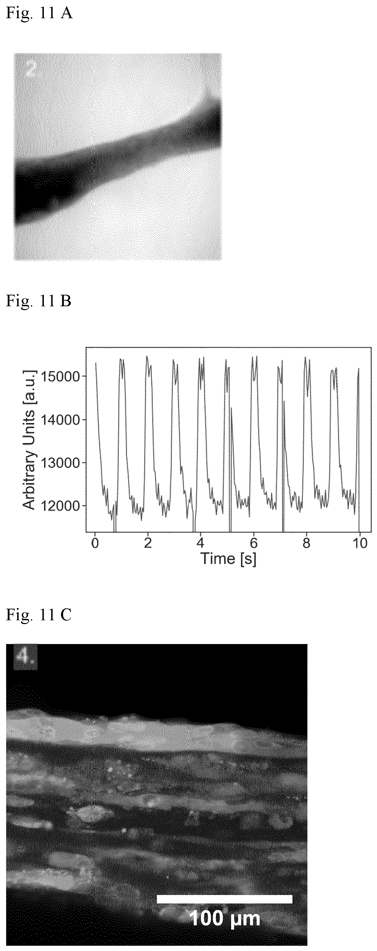

The physical confinement method, on the other hand, produces less homogeneous fibres, but they are still highly controlled in thickness and can yield thin skeletal muscle constructs (see Figure 11A). This method, as opposed to the chemically assisted one, does not require the presence of alginate, (e.g. it does not require a mixture of alginate and gelatin as in the example 1.1) but only requires the presence of gelatin in the cell-laden hydrogel, although at a higher concentration to ensure physical confinement within the polymer coating). In Figure 11A, the hydrogel comprised gelatin at 5% (wt/v) with fibrinogen at 20 mg/mL and myoblasts at a density of 5 million cells/mL. The protocol after co-axial bioprinting was the same as for Example 1.1, except without the addition of EDTA: the fibre constructs were left in a solution of cold thrombin (5 U/mL) for 5 min at 4°C for fibrinogen to crosslink to fibrin and poloxamer 407 to dissolve. After this, additional washes with cold PBS (at 4°C) removed the remaining poloxamer 407 and the constructs were left to proliferate in GM in a cell incubator. As before, the fibers could compact during maturation of the tissue (FIG. 11A) and after several days of differentiation, they could respond to electrical stimulation with contractions, as shown in FIG. 11B. Immunostaining of Myosin Heavy Chain II and cell nuclei revealed thin fibres packed with aligned myotubes (FIG. 11C).

### EXAMPLE 1.3.- Obtaining width-controlled fibers by the physical confinement and chemically assisted physical confinement methods of the invention

For the physical confinement strategy (FIG.12, points 1-2), the hydrogel consisted of 5% (wt/v) of gelatin from porcine skin, type A (Sigma-Aldrich, G2500) and 20 mg/mL of fibrinogen, 3D bioprinted with a co-axial printing method, with Pluronic^{®} F-127 at a concentration of 33% (wt/v).

For the chemical confinement strategy (see FIG.12, points 3-4) a hydrogel consisting of 6% (wt/v) GelMA (CELLINK^{®}, LIK-3050V-1), 1% (wt/v) sodium alginate (Sigma-Aldrich, W201502) and 12 mg/mL of fibrinogen from bovine plasma (Sigma-Aldrich, F8630) was 3D bioprinted with a co-axial printing method, with Pluronic^{®} F-127 powder (Sigma-Aldrich, P2443) at a concentration of 33% (wt/v) and 100 mM of CaCl₂ for the crosslinking of alginate

As is shown in FIG. 12, the fiber diameter can be carefully controlled to the desired dimensions by simply modifying the pressure applied on the hydrogel when printing. For the chemically assisted confinement strategy, the control is finer than for the physical one, achieving diameters that can be as low as the nozzle diameter (200 µm) for 60 kPa of pressure. Under the tested conditions, the physical confinement method, was shown to provide fibers with a mean diameter from 300 µm of width to more than 800 µm, revealing a wider range of possibilities. Lower diameters for each of the methods may be achieved by decreasing further the pressure. It should be noted that, the variability was low, as demonstrated by the error bars, indicating the production of homogeneous fibers (N = 8-12).

### EXAMPLE 2. - Comparative Example: 3D printing by the physical confinement method of the invention vs normal 3D printing

### Example 2.1.- Measuring the width and assessing the homogeneity of the fibers obtained with and without physical confinement

Figure 14 represents the fibre width after 3D bioprinting a biocompatible hydrogel comprising 3.5% (wt/v) gelatin and 20 mg/mL fibrinogen with ("co-axial printing") or without ("normal printing") applying a thermoreversible gelation polymer-based composition coating the biocompatible hydrogel. Poloxamer 407 was dissolved at 33% (wt/v) in ultrapure water under stirring in a refrigerator (4 °C) until fully dissolved. The hydrogel consisted of 3.5% (wt/v) of gelatin from porcine skin, type A (Sigma-Aldrich, G2500) and 20 mg/mL of fibrinogen, 3D bioprinted with a co-axial printing method, with Pluronic^{®} F-127 at a concentration of 33% (wt/v). Firstly, gelatin and HA were dissolved together in PBS at double the desired concentration. Then, fibrinogen was dissolved in PBS at a double concentration and then added to the hydrogel containing gelatin, also at a double concentration, in order to avoid pipetting the viscous mixture.

The obtained results show that with the physical confinement method described herein, fibres are printed individually, and do not fuse with each other due to the protection from the outer polymeric composition, even when several layers are printed on top of each other. The first bar in Figure 14 shows how the width of a co-axial fibre remains around 190 µm both after printing 1 and 3 layers, being 200 µm the diameter of the printing nozzle. This data shows that when using the co-axial method, the fibre width is independent of the number of printed fibres, thus evidencing that there is no fusion between adjacent fibers.

The other two bars show the fibre width of the same hydrogel 3D bioprinted with a standard method. The middle bar shows a single layer of bioink, where the printed fibre reaches a diameter around 3 times larger than that of the co-axial method. Likewise, when three layers are printed on top of each other, the diameter of the fibre increases to more than 1 mm, while the diameter of a co-axial fibre was not affected by the number of fibres printed.

Moreover, the co-axial method provides a finer control of the fibre width, resulting in more homogeneous fibers. Figure 15 shows the standard deviation of a set of 5-7 fibres printed with co-axial or standard method. It can be observed how the standard deviation is significantly smaller for the co-axial method, while for the standard method it remains fairly constant, even when more layers are printed.

### Example 2.2 Measuring the force output of myotube-containing fibers obtained further to differentiation of myoblast-laden fibers obtained with and without physical confinement

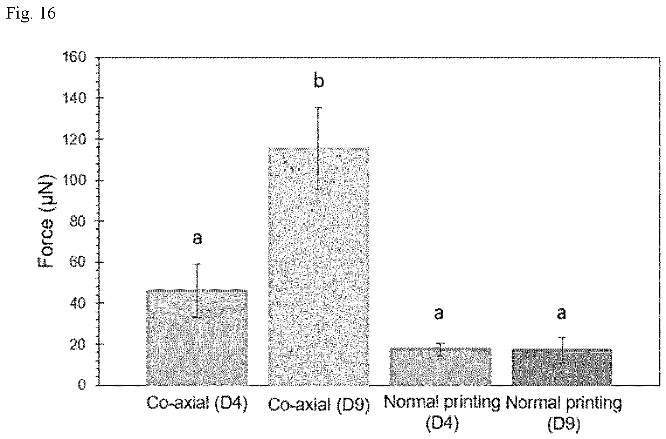

The inventors analysed the force of myotube-containing fiber constructs bioprinted with the co-axial and normal printing methods respectively. The experiment was carried out with C2C12 myoblast concentration of 5 million/mL and a hydrogel composed of 3.5% (wt/v) gelatin and 20 mg/mL fibrinogen. FIG. 16 shows the force output of 5 layers of muscle tissue constructs 3D bioprinted with these two methods and evaluated at day 4 (D4) and day 9 (D9) of differentiation in the differentiation media (DM) and under the culture conditions described herein above.

In particular, tissue constructs 3D bioprinted at day 4 (D4) of differentiation already show a 2-fold improvement with respect to their counterparts bioprinted with the standard method. Moreover, at day 9 (D9) of differentiation, the tissue constructs show a great increment of force, while the standard muscle constructs do not show changes in their force output. The results of the co-axial method at D9 are significantly different from those of the standard method (both D4 and D9), after a t-test with p-value < 0.05.

Accordingly, the results illustrated in FIG.16 show that several layers printed with the physical confinement method of the invention ("co-axial system") produce greater force output than those 3D bioprinted with the standard method.

Without willing to be bound by theory, the observed increase in force can be understood considering the properties of the physical confinement co-axial system of the invention, since the fibres remain individual and not crosslinked (or fused) to each other, as shown in Example 1.1., there are no substantial fusion points between the adjacent fibers when the cell differentiation process is triggered and thus there is increase of the surface area of the fiber which is in contact with the culture medium which facilitates the diffusion of nutrients and oxygen across the structure. This is particularly true especially during the first days of culture. During differentiation of the cells inside the bioink, the fibres usually shrink, and the cells secrete their own extracellular matrix and proteases to remodel the surrounding matrix. This remodelling of the extracellular matrix causes the fibre to get physically closer to adjacent fibers, slightly fusing with the adjacent on certain regions. Accordingly, with the method of the invention there may be some fusion between the fibers in the construct after differentiation but this occurs at a later time point (i.e., during differentiation) and is not particularly dense in the points of contact since there was no original crosslink between adjacent fibers prior to the differentiation step.

To further support these findings, the inventors further measured the degree of fusion between of the myotube containing fibers by using bright field microscopy image and determining changes in intensity which indicate the presence of inhomogeneities in the printed tissue.

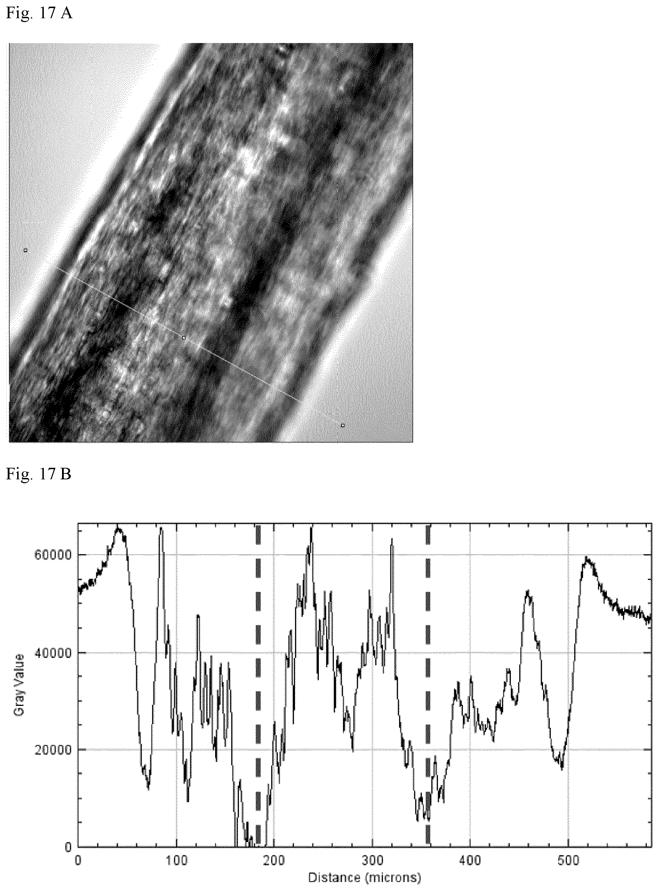

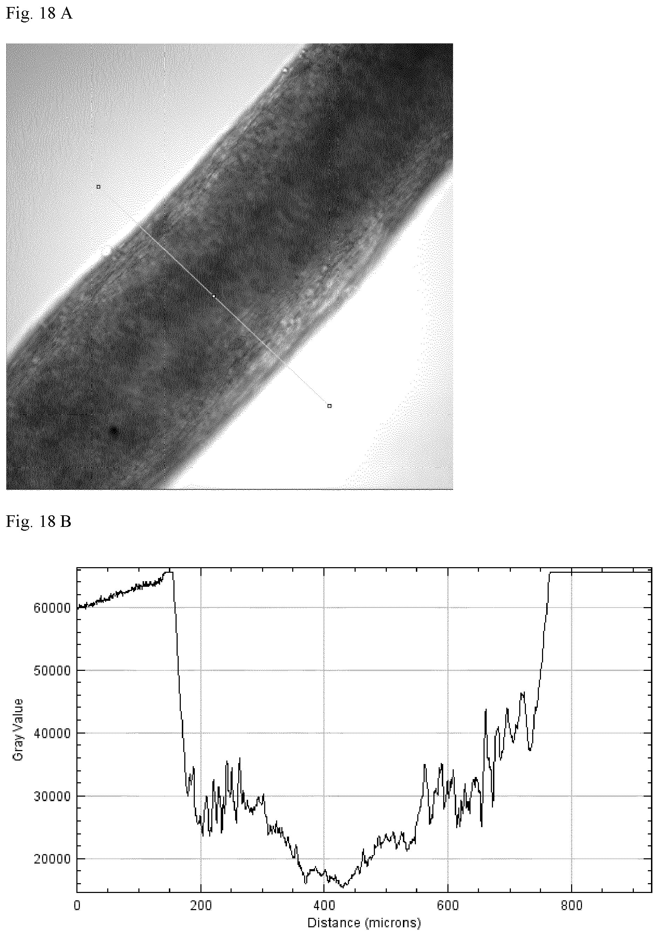

Figure 17A) shows a 3-layer co-axial fibre after 9 days of differentiation. Considering a line perpendicular to the fibre and projecting the intensity values of the bright-field microscopy image (Figure 17B), one can notice inhomogeneities within the tissue construct, consistent with the separation of the 3 layers printed (dashed lines). Likewise, Figure 18A) shows a tissue construct from 3D bioprinting with a standard method. After performing the same intensity projection along a perpendicular line (Figure 18B), one can notice that the intensity distribution is much more homogeneous in this case, indicating that it is a single fibre, with less effective surface area.

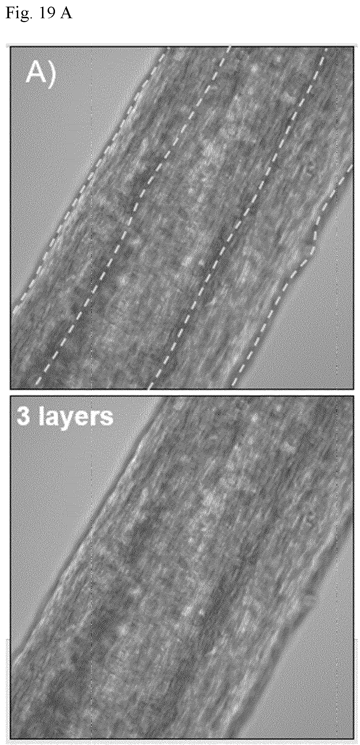

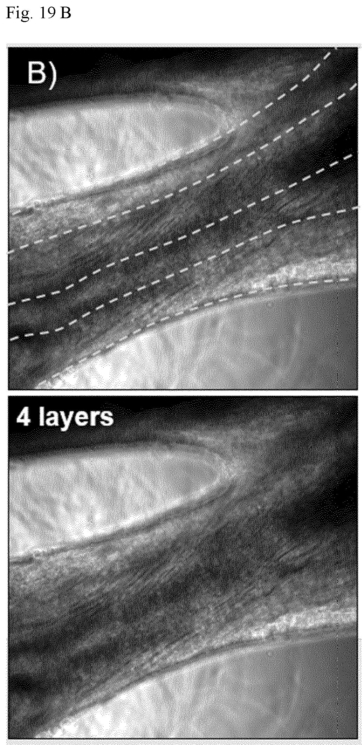

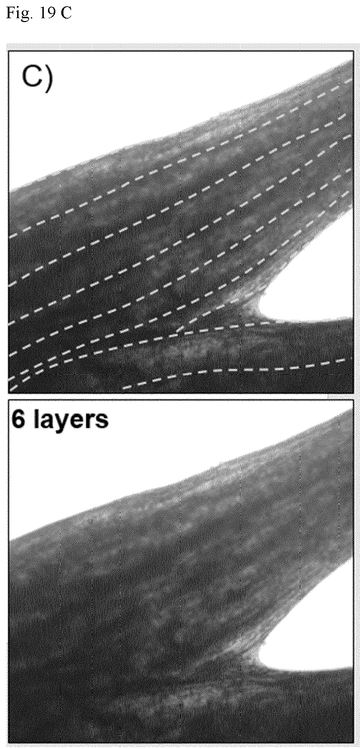

Figure 19 shows more examples of co-axial printing with different number of layers. **In** each case, one can notice the patterns where the fibres fused with each other (indicated by dashed lines). Notice that in several cases, the fusion of the fibres only occurs in specific regions, increasing even further the effective surface area.

All of the above are fully within the scope of the present disclosure and are considered to form the basis for alternative embodiments in which one or more combinations of the above described features are applied, without limitation to the specific combination disclosed above.

## Claims

1. A method for obtaining one or more individual free-form fibers of biocompatible hydrogels with a predefined diameter, wherein said method comprises the use of a printing system comprising at least a first nozzle and a second co-axial nozzle surrounding the first nozzle, wherein said method comprises the following steps:
a) providing a printable biocompatible hydrogel in the first nozzle,
b) providing a printable composition comprising a non-toxic thermoreversible gelation polymer in the second nozzle, wherein said composition comprises or consists of a poloxamer-based thermoreversible gel, and wherein said composition comprises poloxamer 407;
c) extruding the biocompatible hydrogel in a) and the composition in b) simultaneously through the nozzles, wherein the composition in b) in a gel state coats the extruded biocompatible hydrogel;
d) submitting the obtained one or more individual fibers to a cross-linking treatment and
e) removing the composition in b) from the external surface of the deposited one or more fibers ;
wherein
(i) the biocompatible hydrogel comprises a substance which crosslinks upon exposure to a cross-linking agent, and wherein the cross-linking treatment in step d) comprises exposing the obtained fiber to a cross-linking agent; optionally wherein the polymer composition in b) comprises a cross-linking agent and steps c) and d) are performed simultaneously;
(ii) the biocompatible hydrogel comprises a substance which crosslinks upon exposure to UV light, and wherein the cross-linking treatment comprises applying UV light to the obtained fiber; and/or
(iii) the biocompatible hydrogel comprises a substance which crosslinks upon heating, and wherein the cross-linking treatment comprises heating the obtained fiber.

2. The method of claim 1, wherein the cross-linking step d) and the removal of the composition coating the extruded fiber(s) (step e)) are performed at the same time.

3. The method of claim 1 or 2, wherein said fibers are maintained substantially in individual form after deposition in a superposed manner to form a multi-layer construct.

4. The method of any of the preceding claims, wherein the interior of the first nozzle is conical, preferably wherein the first nozzle is of a flexible material, preferably of plastic.

5. The method of any of the preceding claims, wherein a predetermined pressure is applied to the first nozzle, preferably to each nozzle, and
the one or more fibers have a diameter with a standard deviation of 20% or less, preferably 10% or less, more preferably 5% or less with respect to the mean diameter of the fiber(s); and/or
the one or more fibers have a mean diameter with a deviation of 20% or less, preferably 10% or less, more preferably 5% or less with respect to a target diameter, preferably wherein said target diameter is the diameter of the inner nozzle.

6. The method of any of the preceding claims, wherein the composition in b) is in a gel state at a temperature between 20 °C to 25 °C and is removable by wash with an aqueous solution at a temperature between 0 °C and 4 °C, preferably about 4°C.

7. The method of any of the preceding claims, wherein poloxamer 407 is at a concentration from 33% to 40% (wt/v).

8. The method of any of the preceding claims, wherein the biocompatible hydrogel comprises one or more of alginate, modified alginate comprising inserted cell attachment sites, gelatin, fibrinogen, hyaluronic acid, chitosan, poly(ethylene glycol) diacrylate (PEGDA), collagen, nanocellulose, a decellularized extracellular matrix (ECM), protein matrices, ECM proteins, gelatin methacrylate (GelMA), alginate methacrylate (AlgMA), gellan gum, collagen metachrylate (ColMA), agarose, hyaluronic acid methacrylate (HAMA), laminin, xanthan gum or NiPAAM.

9. The method of any of the preceding claims, wherein the biocompatible hydrogel comprises a substance which crosslinks upon exposure to a cross-linking agent, and wherein the cross-linking treatment in step d) comprises exposing the obtained fiber to a cross-linking agent, optionally wherein the polymer composition in b) comprises a cross-linking agent and steps c) and d) are performed simultaneously, wherein:
- the biocompatible hydrogel comprises alginate, modified alginate comprising inserted cell attachment sites or gellan gum, and the cross-linking agent comprises a divalent cation, preferably wherein the cross-linking agent is CaCl₂; and/or
- the biocompatible hydrogel comprises chitosan and the cross-linking agent comprises negatively charged ions, preferably wherein the cross-linking agent comprises negatively charged Molybdenum or Platinum ions.

10. The method of any of the preceding claims, wherein the biocompatible hydrogel comprises a substance which crosslinks enzymatically, and wherein the cross-linking treatment comprises applying an enzymatic cross-linking agent to the obtained fiber, and wherein the biocompatible hydrogel comprises fibrinogen and the enzymatic cross-linking agent is an enzyme solution comprising thrombin.

11. The method of any of the preceding claims, wherein the biocompatible hydrogel comprises a substance which crosslinks upon exposure to UV light, and wherein the cross-linking treatment comprises applying UV light to the obtained fiber; and wherein the biocompatible hydrogel comprises an poly(acrylic) acid hydrogel, such as one or more of gelatin methacrylate (GelMA), diacrylate (PEGDA), or alginate methacrylate (AlgMA) collagen methacrylate (ColMA) or hyaluronan methacrylate (HA-MA).

12. The method of any of the preceding claims, wherein the biocompatible hydrogel comprises a substance which crosslinks upon heating, and wherein the cross-linking treatment comprises heating the obtained fiber; and wherein the biocompatible hydrogel comprises collagen, a decellularized extracellular matrix (ECM) or other protein matrices

13. The method of any of the preceding claims, wherein the biocompatible hydrogel comprises living cells; preferably wherein said living cells are myoblasts, optionally wherein further to the fiber cross-linking and removal of the composition said method further comprises a cell differentiation step, preferably wherein said living cells are myoblasts and differentiate into multi-nucleated myotube structures.

14. An individual free-form fiber of a biocompatible hydrogel wherein said biocompatible hydrogel is as defined in claim 1, wherein said fiber is coated with a composition comprising a thermoreversible gelation polymer in gel state, and wherein said composition comprises poloxamer 407.

15. The individual free-form fiber according to claim 14, wherein the biocompatible hydrogel comprises one or more of gelatin, fibrinogen, hyaluronic acid, chitosan, collagen, nanocellulose, a decellularized extracellular matrix (ECM), protein matrices, a ECM-based hydrogel, ECM proteins, gellan gum, agarose, laminin or xanthan gum.

16. The individual free-form fiber according to claim 15, wherein the biocompatible hydrogel comprises fibrinogen, preferably:
- fibrinogen and gelatin;
- fibrinogen, gelatin and a protein matrix or ECM-based hydrogel.
- fibrinogen, gelatin and hyaluronic acid.

17. The individual free-form fiber according to any of claims 14 to 16, wherein the biological hydrogel comprises living cells, preferably, wherein said living cells are myoblasts; more preferably wherein said myoblasts differentiate into multi-nucleated myotube structures.

18. A multi-layer tissue construct comprising more than one individual fibers as defined in any of claims 14-17 which are superposed to form multiple layers.

19. A hybrid biocompatible machine or biomimetic structure, wherein said hybrid biocompatible machine or biomimetic structure comprises one or more individual fibers as defined in any of claims 14 to 17, preferably wherein said fibers form a multi-layer tissue construct.

20. A method of manufacturing a hybrid biocompatible machine or a biomimetic structure comprising one or more individual fibers of biocompatible hydrogels, wherein said method comprises obtaining one or more individual fibers by the method of any of claims 1 to 13, and wherein said method comprises depositing said one or more individual fibers in step c) on or within said machine or biomimetic structure or assembling these forming a biomimetic structure, preferably wherein said fibers form a multi-layer construct.

21. A printing system for obtaining one or more free-form individual fibers of biocompatible hydrogels with a predefined diameter, wherein the printing system comprises:
- at least a first nozzle and a second co-axial nozzle surrounding the first nozzle;
- a source of a printable biocompatible hydrogel as defined in claim 1 connected to the first nozzle; and
- a source of a non-toxic thermoreversible gelation polymer composition connected to the second nozzle, wherein said composition comprises or consists of a poloxamer-based thermoreversible gel, and wherein said composition comprises poloxamer 407;
wherein the printing system is configured to extrude the biocompatible hydrogel and the non-toxic thermoreversible gelation polymer simultaneously through the nozzles, such that when extruded, the non-toxic polymer coats in a gel state the extruded biocompatible composition.

## Patentansprüche

1. Verfahren zur Gewinnung von einer oder mehreren einzelnen Freiformfasern aus biokompatiblen Hydrogelen mit einem vordefinierten Durchmesser, wobei das genannte Verfahren die Verwendung eines Drucksystems umfasst, welches mindestens eine erste Düse und eine zweite koaxiale Düse, welche die erste Düse umgibt, umfasst, wobei das genannte Verfahren die folgenden Schritte umfasst:
a) das Bereitstellen eines druckbaren biokompatiblen Hydrogels in der ersten Düse,
b) das Bereitstellen einer druckbaren Zusammensetzung, welche ein nichttoxisches thermoreversibles gelbildendes Polymer umfasst, in der zweiten Düse, wobei die genannte Zusammensetzung ein Poloxamerbasiertes thermoreversibles Gel umfasst oder daraus besteht, und wobei die genannte Zusammensetzung Poloxamer 407 umfasst;
c) das Extrudieren des biokompatiblen Hydrogels in a) und der Zusammensetzung in b) gleichzeitig durch die Düsen, wobei die Zusammensetzung in b) im Gelzustand das extrudierte biokompatible Hydrogel beschichtet;
d) das Aussetzen der gewonnenen einen oder mehreren einzelnen Fasern einer Vernetzungsbehandlung und
e) das Entfernen der Zusammensetzung in b) von der äußeren Oberfläche der abgelagerten einen oder mehreren Fasern;
wobei
(i) das biokompatible Hydrogel eine Substanz umfasst, welche sich nach dem Aussetzen einem Vernetzungsmittel vernetzt, und wobei die Vernetzungsbehandlung in Schritt d) das Aussetzen der gewonnenen Faser einem Vernetzungsmittel umfasst; wobei wahlweise die polymere Zusammensetzung in b) ein Vernetzungsmittel umfasst und Schritte c) und d) gleichzeitig durchgeführt werden;
(ii) das biokompatible Hydrogel eine Substanz umfasst, welche sich nach dem Aussetzen einem UV-Licht vernetzt, und wobei die Vernetzungsbehandlung das Anwenden von UV-Licht auf die gewonnene Faser umfasst; und/oder
(iii) das biokompatible Hydrogel eine Substanz umfasst, welche sich nach Erhitzung vernetzt, und wobei die Vernetzungsbehandlung das Erhitzen der gewonnenen Faser umfasst.

2. Verfahren nach Anspruch 1, wobei der Vernetzungsschritt d) und die Beseitigung der Zusammensetzung, welche die extrudierte(n) Faser(n) (Schritt e)) beschichtet, gleichzeitig durchgeführt werden.

3. Verfahren nach Anspruch 1 oder 2, wobei die genannten Fasern im Wesentlichen in Einzelform nach der Ablagerung auf überlagernde Weise aufrechterhalten werden, um ein mehrschichtiges Konstrukt zu bilden.

4. Verfahren nach einem der vorhergehenden Ansprüche, wobei das Innere der ersten Düse konisch ist, wobei vorzugsweise die erste Düse aus einem flexiblen Material, vorzugsweise aus Kunststoff, ist.

5. Verfahren nach einem der vorhergehenden Ansprüche, wobei ein vorbestimmter Druck auf die erste Düse, vorzugsweise auf jede Düse, ausgeübt wird und
die eine oder mehreren Fasern einen Durchmesser mit einer Standardabweichung von 20 % oder weniger, vorzugsweise 10 % oder weniger, weiter vorzugsweise 5 % oder weniger in Bezug auf den mittleren Durchmesser der Faser(n) aufweisen; und/oder
die eine oder mehreren Fasern einen mittleren Durchmesser mit einer Abweichung von 20 % oder weniger, vorzugsweise 10 % oder weniger, weiter vorzugsweise 5 % oder weniger in Bezug auf einen Zieldurchmesser aufweisen, wobei vorzugsweise der genannte Zieldurchmesser der Durchmesser der inneren Düse ist.

6. Verfahren nach einem der vorhergehenden Ansprüche, wobei die Zusammensetzung in b) im Gelzustand bei einer Temperatur zwischen 20 °C und 25 °C ist und durch Auswaschen mit einer wässrigen Lösung bei einer Temperatur zwischen 0 °C und 4 °C, vorzugsweise etwa 4 °C, beseitigt werden kann.

7. Verfahren nach einem der vorhergehenden Ansprüche, wobei das Poloxamer 407 bei einer Konzentration von 33 % bis 40 % (Gew./Vol.) ist.

8. Verfahren nach einem der vorhergehenden Ansprüche, wobei das biokompatible Hydrogel eines oder mehrere aus Alginat, modifiziertem Alginat umfassend eingefügte Zellbindungsstellen, Gelatine, Fibrinogen, Hyaluronsäure, Chitosan, Poly(ethylenglycol)diacrylat (PEGDA), Collagen, Nanocellulose, einer dezellularisierten extrazellulären Matrix (ECM), Proteinmatrizen, ECM-Proteinen, Gelatine-Methacrylat (GelMA), Alginat-Methacrylat (AlgMA), Gellan, Collagen-Methacrylat (ColMA), Agarose, Hyaluronsäure-Methacrylat (HAMA), Laminin, Xanthan oder NiPAAM umfasst.

9. Verfahren nach einem der vorhergehenden Ansprüche, wobei das biokompatible Hydrogel eine Substanz umfasst, welche sich nach dem Aussetzen einem Vernetzungsmittel vernetzt, und wobei die Vernetzungsbehandlung in Schritt d) das Aussetzen der gewonnenen Faser einem Vernetzungsmittel umfasst, wobei wahlweise die polymere Zusammensetzung in b) ein Vernetzungsmittel umfasst und Schritte c) und d) gleichzeitig durchgeführt werden, wobei:
- das biokompatible Hydrogel Alginat, modifiziertes Alginat umfassend eingefügte Zellbindungsstellen oder Gellan umfasst, und das Vernetzungsmittel ein zweiwertiges Kation umfasst, wobei vorzugsweise das Vernetzungsmittel CaCl₂ ist; und/oder
- das biokompatible Hydrogel Chitosan umfasst und das Vernetzungsmittel negativ geladene Ionen umfasst, wobei vorzugsweise das Vernetzungsmittel negativ geladene Molybdän- oder Platin-Ionen umfasst.

10. Verfahren nach einem der vorhergehenden Ansprüche, wobei das biokompatible Hydrogel eine Substanz umfasst, welche sich enzymatisch vernetzt, und wobei die Vernetzungsbehandlung das Anwenden eines enzymatischen Vernetzungsmittels auf die gewonnene Faser umfasst, und wobei das biokompatible Hydrogel Fibrinogen umfasst und das enzymatische Vernetzungsmittel eine Enzymlösung umfassend Thrombin ist.

11. Verfahren nach einem der vorhergehenden Ansprüche, wobei das biokompatible Hydrogel eine Substanz umfasst, welche sich nach dem Aussetzen einem UV-Licht vernetzt, und wobei die Vernetzungsbehandlung das Anwenden von UV-Licht auf die gewonnene Faser umfasst; und wobei das biokompatible Hydrogel ein Polyacrylsäure-Hydrogel, wie eines oder mehrere aus Gelatine-Methacrylat (GelMA), Diacrylat (PEGDA), oder Alginat-Methacrylat (AlgMA), Collagen-Methacrylat (ColMA) oder Hyaluronsäure-Methacrylat (HAMA) umfasst.

12. Verfahren nach einem der vorhergehenden Ansprüche, wobei das biokompatible Hydrogel eine Substanz umfasst, welche sich nach Erhitzung vernetzt, und wobei die Vernetzungsbehandlung das Erhitzen der gewonnenen Faser umfasst; wobei vorzugsweise das biokompatible Hydrogel Collagen, eine dezellularisierte extrazelluläre Matrix (ECM) oder andere Proteinmatrizen umfasst.

13. Verfahren nach einem der vorhergehenden Ansprüche, wobei das biokompatible Hydrogel lebende Zellen umfasst; wobei vorzugsweise die genannten lebenden Zellen Myoblasten sind, wobei wahlweise zusätzlich zur Faservernetzung und zur Beseitigung der Zusammensetzung das genannte Verfahren zusätzlich einen Zelldifferenzierungsschritt umfasst, wobei vorzugsweise die genannten lebenden Zellen Myoblasten sind und sich in mehrkernige Myotubenstrukturen differenzieren.

14. Einzelne Freiformfaser eines biokompatiblen Hydrogels, wobei das genannte biokompatible Hydrogel wie nach Anspruch 1 ist, wobei die genannte Faser mit einer Zusammensetzung beschichtet ist, welche ein thermoreversibles gelbildendes Polymer im Gelzustand umfasst, und wobei die genannte Zusammensetzung Poloxamer 407 umfasst.

15. Einzelne Freiformfaser nach Anspruch 14, wobei das biokompatible Hydrogel eines oder mehrere aus Gelatine, Fibrinogen, Hyaluronsäure, Chitosan, Collagen, Nanocellulose, einer dezellularisierten extrazellulären Matrix (ECM), Proteinmatrizen, einem ECM-basierten Hydrogel, ECM-Proteinen, Gellan, Agarose, Laminin oder Xanthan umfasst.

16. Einzelne Freiformfaser nach Anspruch 15, wobei das biokompatible Hydrogel Fibrinogen umfasst, vorzugsweise:
- Fibrinogen und Gelatine;
- Fibrinogen, Gelatine und eine Proteinmatrix oder ein ECM-basiertes Hydrogel;
- Fibrinogen, Gelatine und Hyaluronsäure.

17. Einzelne Freiformfaser nach einem der Ansprüche 14 bis 16, wobei das biologische Hydrogel lebende Zellen umfasst, wobei vorzugsweise die genannten lebenden Zellen Myoblasten sind; wobei weiter vorzugsweise sich die genannten Myoblasten in mehrkernige Myotubenstrukturen differenzieren.

18. Mehrschichtiges Gewebekonstrukt umfassend mehr als eine einzelne Faser nach einem der Ansprüche 14-17, welche überlagert sind, um mehrfache Schichten zu bilden.

19. Hybride biokompatible Maschine oder biomimetische Struktur, wobei die genannte hybride biokompatible Maschine oder biomimetische Struktur eine oder mehrere einzelne Fasern nach einem der Ansprüche 14 bis 17 umfasst, wobei vorzugsweise die genannten Fasern ein mehrschichtiges Gewebekonstrukt bilden.

20. Verfahren zur Herstellung einer hybriden biokompatiblen Maschine oder einer biomimetischen Struktur umfassend eine oder mehrere einzelne Fasern aus biokompatiblen Hydrogelen, wobei das genannte Verfahren das Gewinnen einer oder mehrerer einzelnen Fasern mittels des Verfahrens nach einem der Ansprüche 1 bis 13 umfasst, und wobei das genannte Verfahren das Ablagern der genannten einen oder mehreren einzelnen Fasern in Schritt c) auf oder innerhalb der genannten Maschine oder biomimetischen Struktur oder das Zusammenfügen derselben unter Bildung einer biomimetischen Struktur umfasst, wobei vorzugsweise die genannten Fasern ein mehrschichtiges Konstrukt bilden.

21. Drucksystem zur Gewinnung von einer oder mehreren einzelnen Freiformfasern aus biokompatiblen Hydrogelen mit einem vordefinierten Durchmesser, wobei das Drucksystem Folgendes umfasst:
- mindestens eine erste Düse und eine zweite koaxiale Düse, welche die erste Düse umgibt;
- eine Quelle von einem druckbaren biokompatiblen Hydrogel nach Anspruch 1, welche mit der ersten Düse verbunden ist; und
- eine Quelle von einer nichttoxischen thermoreversiblen gelbildenden polymeren Zusammensetzung, welche mit der zweiten Düse verbunden ist, wobei die genannte Zusammensetzung ein Poloxamerbasiertes thermoreversibles Gel umfasst oder daraus besteht, und wobei die genannte Zusammensetzung Poloxamer 407 umfasst;
wobei das Drucksystem dazu ausgebildet ist, das biokompatible Hydrogel und das nichttoxische thermoreversible gelbildende Polymer gleichzeitig durch die Düsen zu extrudieren, sodass, wenn sie extrudiert sind, das nichttoxische Polymer im Gelzustand die extrudierte biokompatible Zusammensetzung beschichtet.

## Revendications

1. Procédé pour l'obtention d'une ou plusieurs fibres individuelles de forme libre d'hydrogels biocompatibles ayant un diamètre prédéfini, dans lequel ledit procédé comprend l'utilisation d'un système d'impression comprenant au moins une première buse et une seconde buse coaxiale entourant la première buse, dans lequel ledit procédé comprend les étapes suivantes :
a) fourniture d'un hydrogel biocompatible imprimable au niveau de la première buse,
b) fourniture d'une composition imprimable comprenant un polymère non toxique à gélification thermoréversible au niveau de la seconde buse, dans lequel ladite composition comprend ou est constituée d'un gel thermoréversible à base de poloxamère, et dans lequel ladite composition comprend du poloxamère 407 ;
c) extrusion simultanée de l'hydrogel biocompatible de l'étape a) et de la composition de l'étape b) à travers les buses, dans lequel la composition de l'étape b) à l'état de gel enrobe l'hydrogel biocompatible extrudé ;
d) soumission de l'une ou plusieurs fibres individuelles obtenues à un traitement de réticulation, et
e) enlèvement de la composition de l'étape b) de la surface externe de l'une ou plusieurs fibres déposées ;
dans lequel
(i) l'hydrogel biocompatible comprend une substance qui réticule lors d'une exposition à un agent de réticulation, et dans lequel le traitement de réticulation de l'étape d) comprend l'exposition de la fibre obtenue à un agent de réticulation ; facultativement dans lequel la composition de polymère de l'étape b) comprend un agent de réticulation, et les étapes c) et d) sont effectuées simultanément ;
(ii) l'hydrogel biocompatible comprend une substance qui réticule lors d'une exposition à lumière UV, et dans lequel le traitement de réticulation comprend l'application de lumière UV à la fibre obtenue ; et/ou
(iii) l'hydrogel biocompatible comprend une substance qui réticule lors d'un chauffage, et dans lequel le traitement de réticulation comprend le chauffage de la fibre obtenue.

2. Procédé selon la revendication 1, dans lequel l'étape de réticulation d) et l'enlèvement de la composition enrobant la/les fibre(s) extrudée(s) (étape e)) sont effectués en même temps.

3. Procédé selon la revendication 1 ou 2, dans lequel lesdites fibres sont maintenues sensiblement sous forme individuelle après dépôt de manière superposée afin de former une construction multicouche.

4. Procédé selon l'une quelconque des revendications précédentes, dans lequel l'intérieur de la première buse est conique, de préférence dans lequel la première buse est en matériau souple, de préférence en matière plastique.

5. Procédé selon l'une quelconque des revendications précédentes, dans lequel une pression prédéterminée est appliquée à la première buse, de préférence à chaque buse, et
l'une ou plusieurs fibres présentent un diamètre ayant un écart type de 20 % ou inférieur, de préférence 10 % ou inférieur, plus de préférence 5 % ou inférieur par rapport au diamètre moyen de la/des fibre(s) ; et/ou
l'une ou plusieurs fibres présentent un diamètre moyen ayant un écart de 20 % ou inférieur, de préférence 10 % ou inférieur, plus de préférence 5 % ou inférieur par rapport à un diamètre cible, de préférence dans lequel ledit diamètre cible est le diamètre de la buse interne.

6. Procédé selon l'une quelconque des revendications précédentes, dans lequel la composition de l'étape b) se trouve à l'état de gel à une température comprise entre 20 °C et 25 °C et peut être enlevée par lavage à l'aide d'une solution aqueuse à une température comprise entre 0 °C et 4 °C, de préférence d'environ 4 °C.

7. Procédé selon l'une quelconque des revendications précédentes, dans lequel le poloxamère 407 est présent à une concentration allant de 33 % à 40 % (m/v).

8. Procédé selon l'une quelconque des revendications précédentes, dans lequel l'hydrogel biocompatible comprend un ou plusieurs éléments parmi l'alginate, un alginate modifié comprenant des sites d'attachement cellulaire insérés, la gélatine, le fibrinogène, l'acide hyaluronique, le chitosane, le diacrylate de poly(éthylène glycol) (PEGDA), le collagène, la nanocellulose, une matrice extracellulaire (MEC) décellularisée, des matrices protéiques, des protéines de MEC, le méthacrylate de gélatine (GelMA), le méthacrylate d'alginate (AlgMA), la gomme gellane, le méthacrylate de collagène (ColMA), l'agarose, le méthacrylate d'acide hyaluronique (HAMA), la laminine, la gomme xanthane ou le NiPAAM.

9. Procédé selon l'une quelconque des revendications précédentes, dans lequel l'hydrogel biocompatible comprend une substance qui réticule lors d'une exposition à un agent de réticulation, et dans lequel le traitement de réticulation de l'étape d) comprend l'exposition de la fibre obtenue à un agent de réticulation, facultativement dans lequel la composition de polymère de l'étape b) comprend un agent de réticulation et les étapes c) et d) sont effectuées simultanément, dans lequel :
- l'hydrogel biocompatible comprend de l'alginate, un alginate modifié comprenant de sites d'attachement cellulaire insérés ou de la gomme gellane, et l'agent de réticulation comprend un cation divalent, de préférence dans lequel l'agent de réticulation est le CaCI_{2 ;} et/ou
- l'hydrogel biocompatible comprend du chitosane et l'agent de réticulation comprend des ions chargés négativement, de préférence dans lequel l'agent de réticulation comprend des ions de molybdène ou de platine chargés négativement.

10. Procédé selon l'une quelconque des revendications précédentes, dans lequel l'hydrogel biocompatible comprend une substance qui réticule par voie enzymatique, et dans lequel le traitement de réticulation comprend l'application d'un agent de réticulation enzymatique sur la fibre obtenue, et dans lequel l'hydrogel biocompatible comprend du fibrinogène, et l'agent de réticulation enzymatique est une solution d'enzyme comprenant de la thrombine.

11. Procédé selon l'une quelconque des revendications précédentes, dans lequel l'hydrogel biocompatible comprend une substance qui réticule lors d'une exposition à lumière UV, et dans lequel le traitement de réticulation comprend l'application de lumière UV à la fibre obtenue ; et dans lequel l'hydrogel biocompatible comprend un hydrogel d'acide poly(acrylique), tel qu'un ou plusieurs éléments parmi le méthacrylate de gélatine (GelMA), le diacrylate (PEGDA) ou le méthacrylate d'alginate (AlgMA), le méthacrylate de collagène (ColMA) ou le méthacrylate d'hyaluronane (HA-MA).

12. Procédé selon l'une quelconque des revendications précédentes, dans lequel l'hydrogel biocompatible comprend une substance qui réticule lors d'un chauffage, et dans lequel le traitement de réticulation comprend le chauffage de la fibre obtenue ; et dans lequel l'hydrogel biocompatible comprend du collagène, une matrice extracellulaire (MEC) décellularisée ou d'autres matrices protéiques.

13. Procédé selon l'une quelconque des revendications précédentes, dans lequel l'hydrogel biocompatible comprend des cellules vivantes ; de préférence dans lequel lesdites cellules vivantes sont des myoblastes, facultativement dans lequel, suite à la réticulation de la fibre et l'enlèvement de la composition, ledit procédé comprend en outre une étape de différenciation cellulaire, de préférence dans lequel lesdites cellules vivantes sont des myoblastes et se différencient en structures de myotubes multinucléés.

14. Fibre individuelle de forme libre d'un hydrogel biocompatible dans laquelle ledit hydrogel biocompatible est tel que défini dans la revendication 1, dans laquelle ladite fibre est enrobée d'une composition comprenant un polymère à gélification thermoréversible à l'état de gel, et dans laquelle ladite composition comprend du poloxamère 407.

15. Fibre individuelle de forme libre selon la revendication 14, dans laquelle l'hydrogel biocompatible comprend un ou plusieurs éléments parmi la gélatine, le fibrinogène, l'acide hyaluronique, le chitosane, le collagène, la nanocellulose, une matrice extracellulaire (MEC) décellularisée, des matrices protéiques, un hydrogel à base de MEC, des protéines de MEC, la gomme gellane, l'agarose, la laminine ou la gomme xanthane.

16. Fibre individuelle de forme libre selon la revendication 15, dans laquelle l'hydrogel biocompatible comprend du fibrinogène, de préférence :
- du fibrinogène et de la gélatine ;
- du fibrinogène, de la gélatine et un hydrogel à base de MEC ou matrice protéique ;
- du fibrinogène, de la gélatine et de l'acide hyaluronique.

17. Fibre individuelle de forme libre selon l'une quelconque des revendications 14 à 16, dans laquelle l'hydrogel biologique comprend des cellules vivantes, de préférence, dans laquelle lesdites cellules vivantes sont des myoblastes ; plus de préférence, dans laquelle lesdits myoblastes se différencient en structures de myotubes multinucléés.

18. Construction tissulaire multicouche comprenant plus d'une fibre individuelle telle que définie dans l'une quelconque des revendications 14-17 qui sont superposées pour former des couches multiples.

19. Machine biocompatible hybride ou structure biomimétique, dans laquelle ladite machine biocompatible hybride ou structure biomimétique comprend une ou plusieurs fibres individuelles telles que définies dans l'une quelconque des revendications 14 à 17, de préférence dans laquelle lesdites fibres forment une construction tissulaire multicouche.

20. Procédé de fabrication d'une machine biocompatible hybride ou d'une structure biomimétique comprenant une ou plusieurs fibres individuelles d'hydrogels biocompatibles, dans lequel ledit procédé comprend l'obtention d'une ou plusieurs fibres individuelles par le procédé selon l'une quelconque des revendications 1 à 13, et dans lequel ledit procédé comprend le dépôt desdites une ou plusieurs fibres individuelles de l'étape c) sur ou à l'intérieur de ladite machine ou structure biomimétique, ou l'assemblage de celles-ci de manière à former une structure biomimétique, de préférence dans lequel lesdites fibres forment une construction multicouche.

21. Système d'impression pour l'obtention d'une ou plusieurs fibres individuelles de forme libre d'hydrogels biocompatibles ayant un diamètre prédéfini, dans lequel le système d'impression comprend :
- au moins une première buse et une seconde buse coaxiale entourant la première buse ;
- une source d'un hydrogel biocompatible imprimable tel que défini dans la revendication 1, raccordée à la première buse ; et
- une source d'une composition de polymère non toxique à gélification thermoréversible raccordée à la seconde buse, dans lequel ladite composition comprend ou est constituée d'un gel thermoréversible à base de poloxamère, et dans lequel ladite composition comprend du poloxamère 407 ;
dans lequel le système d'impression est configuré pour extruder l'hydrogel biocompatible et le polymère non toxique à gélification thermoréversible simultanément à travers les buses, de sorte que, lorsqu'il est extrudé, le polymère non toxique enrobe à l'état de gel la composition biocompatible extrudée.
